# EUROPEAN PATENT SPECIFICATION

(11) **EP 1 781 785 B1**
(45) Date of publication and mention of the grant of the patent: **10.11.2010**
(21) Application number: 05792560.4
(22) Date of filing: 20.07.2005
(51) Int. Cl.: C12N 15/10

(54) **METHODS OF PRODUCING MUTANT POLYNUCLEOTIDES**
VERFAHREN ZUR HERSTELLUNG VON MUTIERTEN POLYNUKLEOTIDEN
PROCEDES DE PRODUCTION DE POLYNUCLEOTIDES MUTANTS

(30) Priority: 20.07.2004 US 589502 P; 06.12.2004 US 633756 P
(43) Date of publication of application: 09.05.2007
(60) Divisional application: 09160422.3
(73) Proprietor: Novozymes, Inc., Davis, CA 95616 (US); Novozymes A/S, 2880 Bagsvaerd (DK)
(72) Inventor: HANSEN, Peter, Kamp, DK-4320 Lejre (DK); BJOERNVAD, Mads, Eskelund, DK-2830 Virum (DK); CHERRY, Joel, Davis, CA 95616 (US); JONES, Aubrey, Davis, CA 95616 (US); FISHER, Amanda, Davis, CA 95618 (US)
(74) Representative: Lindum, Peter Würtz
(86) International application number: PCT/US2005/025874
(87) International publication number: WO 2006/017371

(56) References cited:
- LONG A R ET AL: "Isolation and characterisation of smallminded, a Drosophila encoding a new member of the Cdc48p/VCP subfamily of AAA proteins" GENE: AN INTERNATIONAL JOURNAL ON GENES AND GENOMES, ELSEVIER, AMSTERDAM, NL, vol. 208, no. 2, 22 February 1998 (1998-02-22), pages 191-199, XP004114944 ISSN: 0378-1119
- WISMAN E ET AL: "THE BEHAVIOUR OF THE AUTONOMOUS MAIZE TRANSPOSABLE ELEMENT EN/SPM IN ARABIDOPSIS THALIANA ALLOWS EFFICIENT MUTAGENESIS" PLANT MOLECULAR BIOLOGY, SPRINGER, DORDRECHT, NL, vol. 37, no. 6, August 1998 (1998-08), pages 989-999, XP001180937 ISSN: 0167-4412
- COHEN G H ET AL: "EXPRESSION OF HERPES SIMPLEX VIRUS TYPE 1 GLYCOPROTEIN D DELETION MULTANTS IN MAMMALIAN CELLS" JOURNAL OF VIROLOGY, NEW YORK, US, US, vol. 62, no. 8, June 1988 (1988-06), pages 1932-1940, XP002905690 ISSN: 0022-538X
- CLANCY A ET AL: "Construction and characterization of transposon TnphoZ for the identification of genes encoding exported proteins in Streptococcus agalactiae" FEMS MICROBIOLOGY LETTERS, AMSTERDAM, NL, vol. 241, no. 2, 15 December 2004 (2004-12-15), pages 257-264, XP004681845 ISSN: 0378-1097

### SEQUENCE LISTING

<110> Novozymes, Inc.
   Novozymes A/S
<120> Methods of producing mutant polynucleotides
<130> 10655.504-WO
<150> 60/589,502
   <151> 2004-07-20
<150> 60/633,756
   <151> 2004-12-06
<160> 21
<170> PatentIn version 3.2
<210> 1
   <211> 2586
   <212> DNA
   <213> Aspergillus oryzae
<400> 1
   atgaagcttg gttggatcga ggtggccgca ttggcggctg cctcagtagt cagtgccaag      60
   gatgatctcg cgtactcccc tcctttctac ccttccccat gggcagatgg tcagggtgaa     120
   tgggcggaag tatacaaacg cgctgtagac atagtttccc agatgacgtt gacagagaaa     180
   gtcaacttaa cgactggaac aggatggcaa ctagagaggt gtgttggaca aactggcagt     240
   gttcccagac tcaacatccc cagcttgtgt ttgcaggata gtcctcttgg tattcgtttc     300
   tcggactaca attcagcttt ccctgcgggt gttaatgtcg ctgccacctg ggacaagacg     360
   ctcgcctacc ttcgtggtca ggcaatgggt gaggagttca gtgataaggg tattgacgtt     420
   cagctgggtc ctgctgctgg ccctctcggt gctcatccgg atggcggtag aaactgggaa     480
   ggtttctcac cagatccagc cctcaccggt gtactttttg cggagacgat taagggtatt     540
   caagatgctg gtgtcattgc gacagctaag cattatatca tgaacgaaca agagcatttc     600
   cgccaacaac ccgaggctgc gggttacgga ttcaacgtaa gcgacagttt gagttccaac     660
   gttgatgaca agactatgca tgaattgtac ctctggccct tcgcggatgc agtacgcgct     720
   ggagtcggtg ctgtcatgtg ctcttacaac caaatcaaca acagctacgg ttgcgagaat     780
   agcgaaactc tgaacaagct tttgaaggcg gagcttggtt tccaaggctt cgtcatgagt     840
   gattggaccg ctcatcacag cggcgtaggc gctgctttag caggtctgga tatgtcgatg     900
   cccggtgatg ttaccttcga tagtggtacg tctttctggg gtgcaaactt gacggtcggt     960
   gtccttaacg gtacaatccc ccaatggcgt gttgatgaca tggctgtccg tatcatggcc    1020
   gcttattaca aggttggccg cgacaccaaa tacacccctc ccaacttcag ctcgtggacc    1080
   agggacgaat atggtttcgc gcataaccat gtttcggaag gtgcttacga gagggtcaac   1140
   gaattcgtgg acgtgcaacg cgatcatgcc gacctaatcc gtcgcatcgg cgcgcagagc   1200
   actgttctgc tgaagaacaa gggtgccttg cccttgagcc gcaaggaaaa gctggtcgcc   1260
   cttctgggag aggatgcggg ttccaactcg tggggcgcta acggctgtga tgaccgtggt   1320
   tgcgataacg gtacccttgc catggcctgg ggtagcggta ctgcgaattt cccatacctc   1380
   gtgacaccag agcaggcgat tcagaacgaa gttcttcagg gccgtggtaa tgtcttcgcc   1440
   gtgaccgaca gttgggcgct cgacaagatc gctgcggctg cccgccaggc cagcgtatct   1500
   ctcgtgttcg tcaactccga ctcaggagaa ggctatctta gtgtggatgg aaatgagggc   1560
   gatcgtaaca acatcactct gtggaagaac ggcgacaatg tggtcaagac cgcagcgaat   1620
   aactgtaaca acaccgttgt catcatccac tccgtcggac cagttttgat cgatgaatgg   1680
   tatgaccacc ccaatgtcac tggtattctc tgggctggtc tgccaggcca ggagtctggt   1740
   aactccattg ccgatgtgct gtacggtcgt gtcaaccctg gcgccaagtc tcctttcact   1800
   tggggcaaga cccgggagtc gtatggttct cccttggtca aggatgccaa caatggcaac   1860
   ggagcgcccc agtctgattt cacccagggt gttttcatcg attaccgcca tttcgataag   1920
   ttcaatgaga cccctatcta cgagtttggc tacggcttga gctacaccac cttcgagctc   1980
   tccgacctcc atgttcagcc cctgaacgcg tcccgataca ctcccaccag tggcatgact   2040
   gaagctgcaa agaactttgg tgaaattggc gatgcgtcgg agtacgtgta tccggagggg   2100
   ctggaaagga tccatgagtt tatctatccc tggatcaact ctaccgacct gaaggcatcg   2160
   tctgacgatt ctaactacgg ctgggaagac tccaagtata ttcccgaagg cgccacggat   2220
   gggtctgccc agccccgttt gcccgctagt ggtggtgccg gaggaaaccc cggtctgtac   2280
   gaggatcttt tccgcgtctc tgtgaaggtc aagaacacgg gcaatgtcgc cggtgatgaa   2340
   gttcctcagc tgtacgtttc cctaggcggc ccgaatgagc ccaaggtggt actgcgcaag   2400
   tttgagcgta ttcacttggc cccttcgcag gaggccgtgt ggacaacgac ccttacccgt   2460
   cgtgaccttg caaactggga cgtttcggct caggactgga ccgtcactcc ttaccccaag   2520
   acgatctacg ttggaaactc ctcacggaaa ctgccgctcc aggcctcgct gcctaaggcc   2580
   cagtaa                                                              2586
<210> 2
   <211> 861
   <212> PRT
   <213> Aspergillus oryzae
<400> 2
   Met Lys Leu Gly Trp Ile Glu Val Ala Ala Leu Ala Ala Ala Ser Val
   1               5                   10                  15
   Val Ser Ala Lys Asp Asp Leu Ala Tyr Ser Pro Pro Phe Tyr Pro Ser
               20                  25                  30
   Pro Trp Ala Asp Gly Gln Gly Glu Trp Ala Glu Val Tyr Lys Arg Ala
           35                  40                  45
   Val Asp Ile Val Ser Gln Met Thr Leu Thr Glu Lys Val Asn Leu Thr
       50                  55                  60
   Thr Gly Thr Gly Trp Gln Leu Glu Arg Cys Val Gly Gln Thr Gly Ser
   65                  70                  75                  80
   Val Pro Arg Leu Asn Ile Pro Ser Leu Cys Leu Gln Asp Ser Pro Leu
                   85                  90                  95
   Gly Ile Arg Phe Ser Asp Tyr Asn Ser Ala Phe Pro Ala Gly Val Asn
               100                 105                 110
   Val Ala Ala Thr Trp Asp Lys Thr Leu Ala Tyr Leu Arg Gly Gln Ala
           115                 120                 125
   Met Gly Glu Glu Phe Ser Asp Lys Gly Ile Asp Val Gln Leu Gly Pro
       130                 135                 140
   Ala Ala Gly Pro Leu Gly Ala His Pro Asp Gly Gly Arg Asn Trp Glu
   145                 150                 155                 160
   Gly Phe Ser Pro Asp Pro Ala Leu Thr Gly Val Leu Phe Ala Glu Thr
                   165                 170                 175
   Ile Lys Gly Ile Gln Asp Ala Gly Val Ile Ala Thr Ala Lys His Tyr
               180                 185                 190
   Ile Met Asn Glu Gln Glu His Phe Arg Gln Gln Pro Glu Ala Ala Gly
           195                 200                 205
   Tyr Gly Phe Asn Val Ser Asp Ser Leu Ser Ser Asn Val Asp Asp Lys
       210                 215                 220
   Thr Met His Glu Leu Tyr Leu Trp Pro Phe Ala Asp Ala Val Arg Ala
   225                 230                 235                 240
   Gly Val Gly Ala Val Met Cys Ser Tyr Asn Gln Ile Asn Asn Ser Tyr
   245 250 255
   Gly Cys Glu Asn Ser Glu Thr Leu Asn Lys Leu Leu Lys Ala Glu Leu
   260 265 270
   Gly Phe Gln Gly Phe Val Met Ser Asp Trp Thr Ala His His Ser Gly
   275 280 285
   Val Gly Ala Ala Leu Ala Gly Leu Asp Met Ser Met Pro Gly Asp Val
   290 295 300
   Thr Phe Asp Ser Gly Thr Ser Phe Trp Gly Ala Asn Leu Thr Val Gly
   305 310 315 320
   Val Leu Asn Gly Thr Ile Pro Gln Trp Arg Val Asp Asp Met Ala Val
   325 330 335
   Arg Ile Met Ala Ala Tyr Tyr Lys Val Gly Arg Asp Thr Lys Tyr Thr
   340 345 350
   Pro Pro Asn Phe Ser Ser Trp Thr Arg Asp Glu Tyr Gly Phe Ala His
   355 360 365
   Asn His Val Ser Glu Gly Ala Tyr Glu Arg Val Asn Glu Phe Val Asp
   370 375 380
   Val Gln Arg Asp His Ala Asp Leu Ile Arg Arg Ile Gly Ala Gln Ser
   385 390 395 400
   Thr Val Leu Leu Lys Asn Lys Gly Ala Leu Pro Leu Ser Arg Lys Glu
   405 410 415
   Lys Leu Val Ala Leu Leu Gly Glu Asp Ala Gly Ser Asn Ser Trp Gly
   420 425 430
   Ala Asn Gly Cys Asp Asp Arg Gly Cys Asp Asn Gly Thr Leu Ala Met
   435 440 445
   Ala Trp Gly Ser Gly Thr Ala Asn Phe Pro Tyr Leu Val Thr Pro Glu
   450 455 460
   Gln Ala Ile Gln Asn Glu Val Leu Gln Gly Arg Gly Asn Val Phe Ala
   465 470 475 480
   Val Thr Asp Ser Trp Ala Leu Asp Lys Ile Ala Ala Ala Ala Arg Gln
                   485                 490                 495
   Ala Ser Val Ser Leu Val Phe Val Asn Ser Asp Ser Gly Glu Gly Tyr
               500                 505                 510
   Leu Ser Val Asp Gly Asn Glu Gly Asp Arg Asn Asn Ile Thr Leu Trp
           515                 520                 525
   Lys Asn Gly Asp Asn Val Val Lys Thr Ala Ala Asn Asn Cys Asn Asn
       530                 535                 540
   Thr Val Val Ile Ile His Ser Val Gly Pro Val Leu Ile Asp Glu Trp
   545                 550                 555                 560
   Tyr Asp His Pro Asn Val Thr Gly Ile Leu Trp Ala Gly Leu Pro Gly
                   565                 570                 575
   Gln Glu Ser Gly Asn Ser Ile Ala Asp Val Leu Tyr Gly Arg Val Asn
               580                 585                 590
   Pro Gly Ala Lys Ser Pro Phe Thr Trp Gly Lys Thr Arg Glu Ser Tyr
           595                 600                 605
   Gly Ser Pro Leu Val Lys Asp Ala Asn Asn Gly Asn Gly Ala Pro Gln
       610                 615                 620
   Ser Asp Phe Thr Gln Gly Val Phe Ile Asp Tyr Arg His Phe Asp Lys
   625                 630                 635                 640
   Phe Asn Glu Thr Pro Ile Tyr Glu Phe Gly Tyr Gly Leu Ser Tyr Thr
                   645                 650                 655
   Thr Phe Glu Leu Ser Asp Leu His Val Gln Pro Leu Asn Ala Ser Arg
               660                 665                 670
   Tyr Thr Pro Thr Ser Gly Met Thr Glu Ala Ala Lys Asn Phe Gly Glu
           675                 680                 685
   Ile Gly Asp Ala Ser Glu Tyr Val Tyr Pro Glu Gly Leu Glu Arg Ile
       690                 695                 700
   His Glu Phe Ile Tyr Pro Trp Ile Asn Ser Thr Asp Leu Lys Ala Ser
   705                 710                 715                 720
   Ser Asp Asp Ser Asn Tyr Gly Trp Glu Asp Ser Lys Tyr Ile Pro Glu
   725 730 735
   Gly Ala Thr Asp Gly Ser Ala Gln Pro Arg Leu Pro Ala Ser Gly Gly
   740 745 750
   Ala Gly Gly Asn Pro Gly Leu Tyr Glu Asp Leu Phe Arg Val Ser Val
   755 760 765
   Lys Val Lys Asn Thr Gly Asn Val Ala Gly Asp Glu Val Pro Gln Leu
   770 775 780
   Tyr Val Ser Leu Gly Gly Pro Asn Glu Pro Lys Val Val Leu Arg Lys
   785 790 795 800
   Phe Glu Arg Ile His Leu Ala Pro Ser Gln Glu Ala Val Trp Thr Thr
   805 810 815
   Thr Leu Thr Arg Arg Asp Leu Ala Asn Trp Asp Val Ser Ala Gln Asp
   820 825 830
   Trp Thr Val Thr Pro Tyr Pro Lys Thr Ile Tyr Val Gly Asn Ser Ser
   835 840 845
   Arg Lys Leu Pro Leu Gln Ala Ser Leu Pro Lys Ala Gln
   850 855 860
<210> 3
   <211> 32
   <212> DNA
   <213> Aspergillus oryzae
<400> 3
   gcagatctac catgaagctt ggttggatcg ag 32
<210> 4
   <211> 30
   <212> DNA
   <213> Aspergillus oryzae
<400> 4
   gcctcgagtt actgggcctt aggcagcgag 30
<210> 5
   <211> 36
   <212> DNA
   <213> Aspergillus oryzae
<400> 5
   gcacgcgtcg acaccatgaa gcttggttgg atcgag 36
<210> 6
   <211> 24
   <212> DNA
   <213> Aspergillus oryzae
<400> 6
   gatgcacatg actcgagtta ctgg 24
<210> 7
   <211> 59
   <212> DNA
   <213> Artificial Sequence
<220>
   <223> Primer
<400> 7
   tcgagatcga acagcggccg catcgcagct ggcaggtacg gatcgatcct agtaagcca 59
<210> 8
   <211> 88
   <212> DNA
   <213> Artificial Sequence
<220>
   <223> Primer
<220>
   <221> misc_feature
   <222> (35)..(37)
   <223> n is a, c, g, or t
<400> 8
   gaataaatcg aactggatca tccgacctgc tcgannnagc tcgactgcct tagctgcacg 60
   tgcagatgcg gccgctgagc tcgatcgt 88
<210> 9
   <211> 1139
   <212> DNA
   <213> Artificial Sequence
<220>
   <223> Primer
<400> 9
   gatctgcggc cgcgcacgaa aaacgcgaaa gcgtttcacg ataaatgcga aaacggatcg 60
   atcctagtaa gccacgttgt gtctcaaaat ctctgatgtt acattgcaca agataaaaat 120
   atatcatcat gaacaataaa actgtctgct tacataaaca gtaatacaag gggtgttatg 180
   agccatattc aacgggaaac gtcttgctcg aggccgcgat taaattccaa catggatgct     240
   gatttatatg ggtataaatg ggctcgcgat aatgtcgggc aatcaggtgc gacaatctat     300
   cgattgtatg ggaagcccga tgcgccagag ttgtttctga aacatggcaa aggtagcgtt     360
   gccaatgatg ttacagatga gatggtcaga ctaaactggc tgacggaatt tatgcctctt     420
   ccgaccatca agcattttat ccgtactcct gatgatgcat ggttactcac cactgcgatc     480
   cccgggaaaa cagcattcca ggtattagaa gaatatcctg attcaggtga aaatattgtt     540
   gatgcgctgg cagtgttcct gcgccggttg cattcgattc ctgtttgtaa ttgtcctttt     600
   aacagcgatc gcgtatttcg tctcgctcag gcgcaatcac gaatgaataa cggtttggtt     660
   gatgcgagtg attttgatga cgagcgtaat ggctggcctg ttgaacaagt ctggaaagaa     720
   atgcataagc ttttgccatt ctcaccggat tcagtcgtca ctcatggtga tttctcactt     780
   gataacctta tttttgacga ggggaaatta ataggttgta ttgatgttgg acgagtcgga     840
   atcgcagacc gataccagga tcttgccatc ctatggaact gcctcggtga gttttctcct     900
   tcattacaga aacggctttt tcaaaaatat ggtattgata atcctgatat gaataaattg     960
   cagtttcatt tgatgctcga tgagtttttc taatcagaat tggttaattg gttgtaacac    1020
   tggcagagca ttacgctgac ttgacgggac ggcggctttg ttgaataaat cgaactggat    1080
   catccgtttt cgcatttatc gtgaaacgct ttcgcgtttt tcgtgcgcgg ccgcagatc     1139
<210> 10
   <211> 1111
   <212> DNA
   <213> Artificial Sequence
<220>
   <223> Entranceposon(TM) with DNA fragment inserted comprising outside cutter sites and random or partially random codon triplet 'NNN'; see Figure 1 for details.
<220>
   <221> misc_feature
   <222> (1070)..(1072)
   <223> n is a, c, g, or t
<400> 10
   agcggccgca tcgcagctgg caggtacgga tcgatcctag taagccacgt tgtgtctcaa      60
   aatctctgat gttacattgc acaagataaa aatatatcat catgaacaat aaaactgtct     120
   gcttacataa acagtaatac aaggggtgtt atgagccata ttcaacggga aacgtcttgc     180
   tcgaggccgc gattaaattc caacatggat gctgatttat atgggtataa atgggctcgc     240
   gataatgtcg ggcaatcagg tgcgacaatc tatcgattgt atgggaagcc cgatgcgcca     300
   gagttgtttc tgaaacatgg caaaggtagc gttgccaatg atgttacaga tgagatggtc 360
   agactaaact ggctgacgga atttatgcct cttccgacca tcaagcattt tatccgtact 420
   cctgatgatg catggttact caccactgcg atccccggga aaacagcatt ccaggtatta 480
   gaagaatatc ctgattcagg tgaaaatatt gttgatgcgc tggcagtgtt cctgcgccgg 540
   ttgcattcga ttcctgtttg taattgtcct tttaacagcg atcgcgtatt tcgtctcgct 600
   caggcgcaat cacgaatgaa taacggtttg gttgatgcga gtgattttga tgacgagcgt 660
   aatggctggc ctgttgaaca agtctggaaa gaaatgcata agcttttgcc attctcaccg 720
   gattcagtcg tcactcatgg tgatttctca cttgataacc ttatttttga cgaggggaaa 780
   ttaataggtt gtattgatgt tggacgagtc ggaatcgcag accgatacca ggatcttgcc 840
   atcctatgga actgcctcgg tgagttttct ccttcattac agaaacggct ttttcaaaaa 900
   tatggtattg ataatcctga tatgaataaa ttgcagtttc atttgatgct cgatgagttt 960
   ttctaatcag aattggttaa ttggttgtaa cactggcaga gcattacgct gacttgacgg 1020
   gacggcggct ttgttgaata aatcgaactg gatcatccga cctgctcgan nnagctcgac 1080
   tgccttagct gcacgtgcag atgcggccgc t 1111
<210> 11
   <211> 85
   <212> DNA
   <213> Aspergillus oryzae
<220>
   <221> misc_feature
   <222> (47)..(49)
   <223> n is a, c, g, or t
<400> 11
   gaataaatcg aactggatca tccgctgaag tgcagccact tggacgnnna accaattggg 60
   tgcagaagcg gccgctgagc tcgat 85
<210> 12
   <211> 53
   <212> DNA
   <213> Aspergillus oryzae
<400> 12
   tcgagatcga acagcggccg ctggacttca gacggatcga tcctagtaag cca 53
<210> 13
   <211> 181
   <212> DNA
   <213> Aspergillus oryzae
<220>
   <221> misc_feature
   <222> (88)..(90)
   <223> n=a,c,g,t
<220>
   <221> misc_feature
   <222> (158)..(160)
   <223> n is a, c, g, or t
<400> 13
   tgcggccgct ggacttcaga cggatcgatc ctagtaagcc agaataaatc gaactggatc 60
   atccgctgaa gtgcagccac ttggacgnnn aaccaattgg gtgcagaagc gcttatttag 120
   cttgacctag taggcgactt cacgtcggtg aacctgcnnn ttggttaacc cacgtcttcg 180
   c 181
<210> 14
   <211> 48
   <212> DNA
   <213> Artificial sequence
<220>
   <223> Primer
<400> 14
   gaataaatcg aactggatca tccggtgcag gcggccgctg agctcgat 48
<210> 15
   <211> 55
   <212> DNA
   <213> Artificial Sequence
<220>
   <223> Primer
<400> 15
   tcgagatcga acagcggccg caaggaactg cacacggatc gatcctagta agcca 55
<210> 16
   <211> 82
   <212> DNA
   <213> Artificial Sequence
<220>
   <223> Primer
<400> 16
   tgcggccgca aggaactgca cacggatcga tcctagtaag ccagaataaa tcgaactgga 60
   tcatccggtg caggcggccg ca 82
<210> 17
   <211> 88
   <212> DNA
   <213> Artifiicial Sequence
<220>
   <221> misc_feature
   <222> (52)..(54)
   <223> n is a, c, g, or t
<400> 17
   acgatcgagc tcagcggccg catctgcacg tgcagctaag gcagtcgagc tnnntcgagc 60
   aggtcggatg atccagttcg atttattc 88
<210> 18
   <211> 85
   <212> DNA
   <213> Artificial Sequence
<220>
   <223> Primer
<220>
   <221> misc_feature
   <222> (37)..(39)
   <223> n=a,c,g,t
<400> 18
   atcgagctca gcggccgctt ctgcacccaa ttggttnnnc gtccaagtgg ctgcacttca 60
   gcggatgatc cagttcgatt tattc 85
<210> 19
   <211> 48
   <212> DNA
   <213> Artificial Sequence
<220>
   <223> Primer
<400> 19
   atcgagctca gcggccgcct gcaccggatg atccagttcg atttattc 48
<210> 20
   <211> 52
   <212> DNA
   <213> Artificial Sequence
<220>
   <223> Primer
<400> 20
   tcgagatcga acagcggccg caagctgcac acggatcgat cctagtaagc ca 52
<210> 21
   <211> 49
   <212> DNA
   <213> Articial Sequence
<400> 21
   tcgagatcga acagcggccg cctgcacacg gatcgatcct agtaagcca 49

## Description

### Background of the Invention

### Field of the Invention

The present invention relates to methods of producing mutants of a polynucleotide and to mutant polynucleotides and artificial variants encoded by the mutant polynucleotides.

### Description of the Related Art

The diversity necessary for screening in directed evolution of proteins is often created by error prone mutagenesis to find mutations or positions influencing enzyme activity. Although error prone mutagenesis in principle mutates all base pairs randomly, the outcome of the mutagenesis is rather limited for two main reasons: (A) a given amino acid codon is typically mutated to only 6 or 7 other residues (from one substitution per codon, two or three substitutions are very unlikely), and (B) the mutation rate is biased towards A-T base pairs. Typically 75% of the mutated base pairs are A-T pairs, leaving only 25% of mutated G-C pairs, and the resulting mutation is also biased towards certain bases. Also, additional mutations are normally included to overcome silent mutations, which enhance the chance of hitting destructive mutations due to error in folding, maturation, secretion, etc.

Transposons are segments of DNA that can move around to different positions in the genome of a single cell. They can cause mutations and/or an increase (or decrease) in the amount of DNA in the genome. These mobile segments of DNA are sometimes called "jumping genes".

Many transposons move by a "cut and paste" process. The transposon is cut out of its location and inserted into a new location. This process requires a transposase that is encoded within some transposons. Transposase binds to both ends of the transposon, which consists of inverted repeats which are identical sequences reading in opposite directions, and to a sequence of DNA that makes up the target site. Some transposases require a specific sequence as their target site while others can insert the transposon anywhere in the genome. The DNA at the target site is cut in an offset manner, like the "sticky ends" produced by some restriction enzymes. After the transposon is ligated to the host DNA, the gaps are filled in by Watson-Crick base pairing, which creates identical direct repeats at each end of the transposon.

Often transposons lose their gene for transposase, but as long as there is a transposon in the cell that can synthesize the enzyme, their inverted repeats are recognized and they, too, can be moved to a new location. Alternatively, if it desirable that the transposon remains stably integrated in the same place, the transposase may be provided transiently *in trans,* which is often the case when *in vitro* transposition is carried out.

Transposons have proven to be invaluable genetic tools for molecular geneticists. Several uses of transposons include mutagenesis for gene identification, reporter libraries for analysis of gene expression, and DNA sequencing for relative gene positioning on genetic maps. Until recently, however, all of these applications involved the use of *in vivo* transposition reactions. However, the commercialization of several *in vitro* transposition reactions for DNA sequencing and mutagenesis could lead to the replacement of these more traditional *in vivo* methodologies with more efficient biochemical procedures.

The use of *in vitro* transposition for the mutagenesis of specific genes was first reported by Gwinn et al., 1997, Journal of Bacteriology 179: 7315-7320, where genomic DNA from a naturally transformable microorganism (*Haemophilus influenzae*) was mutagenized using the Tn7 *in vitro* transposition system. DNA sequencing using primers that hybridize to the end of the transposon identified mutations in the genes resulting in a reduced expression of constitutive competence genes.

Reich et al., 1999, Journal of Bacteriology 181: 4961-4968, disclose the use of the Ty1-based transposition system (Primer Island) to scan the entire *Haemophilus influenzae* genome for essential genes. Essential genes were identified by two methods: mutation exclusion and zero time analysis. Mutational exclusion involves the identification of open reading frames that do not contain transposon insertions. Zero time analysis involves the monitoring of the growth of individual cells after transformations over time.

U.S. Patent No. 6,673,567 discloses methods for identifying genes, open reading frames, and other nucleic acid molecules which are essential for the expression of a specific phenotype in microorganisms. The method employs *in vitro* transposition in conjunction with a chromosomal integration vector containing a specific gene or genetic element whose function is unknown. Subsequent transformation of a recombination proficient host with the vector and growth first under non-integrating conditions and then under integrating conditions, followed by a selection screen for either single or double crossover events, results in transformants that may be subjected to phenotypic screens to determine gene function.

U.S. Patent No. 6,562,624 discloses methods for facilitating site-directed homologous recombination in a eukaryotic organism to produce genomic mutants using transposon-mediated mutagenesis of cosmid vectors carrying large genomic inserts from the target eukaryotic organism. The transposon carries a bifunctional marker that can be used for selection in both bacteria and the target eukaryotic organism. Minimization of the length of the cosmid vector allows for maximization of the size of the genomic insert carried by the cosmid. Maximization of the size of the genomic insert increases the frequency of homologous recombination with the genome of the target eukaryotic organism.

The present transposon-based mutagenesis technology is limited in its application because there is no differentiation between mutants in which a transposon has inserted into target DNA versus mutants that have the transposon inserted into adjacent, non-target DNA such as plasmid vector sequences. Previously, to create a mutagenic library that contained only clones in which the transposon was targeted to the desired DNA sequence required excision, purification, and subcloning of those target DNA's containing a transposon. There is a need in the art for a simplified method of subcloning transposon-containing targeted DNA in a single step.

Applying transposon technology combined with outside cutters (restriction endonucleases cutting outside their recognition sequence), it is possible to produce a polypeptide library with one or more substituted amino acids. For instance, an amino acid in a position may be substituted to provide a polypeptide library including each of the remaining 20 natural amino acids in that position. Applying transposon technology and outsite cutters, it is also possible to produce polypeptide libraries with insertions or deletions: in theory any number of coding triplets can be inserted, and with the outside cutters presently known up to 5 triplets can be deleted, but this number may increase with the discovery of new outside cutters that cut farther away from their recognition sequence than the ones presently known.

The object of the present invention is to provide new methods of producing mutant polynucleotides.

### Summary of the Invention

The present invention relates to methods of producing at least one mutant of a polynucleotide, the method comprising the steps of:
(a) isolating a first library of constructs, wherein each construct comprises a first selectable marker, a polynucleotide, an inserted artificial transposon comprising at least two restriction endonuclease recognition sites and a second selectable marker, and a first recombination site flanking the 5' end of the polynucleotide and a second recombination site flanking the 3' end of the polynucleotide, wherein the artificial transposon has inserted at one or more random sites within the constructs, and wherein the first library is selected using the first and second selectable markers in a first host cell;
(b) isolating a second library of constructs by introducing the first library of constructs into a vector comprising a third selectable marker and a first recombination site and a second recombination site to facilitate site-specific recombination of the first recombination site flanking the 5' end of the polynucleotide and the second recombination site flanking the 3' end of the polynucleotide in the first library of constructs with the first recombination site and the second recombination site of the vector and by selecting the second library of constructs using the second and third selectable markers in a second host cell;
(c) isolating an insertion library containing at least one substitution, deletion, or insertion of at least one nucleotide in each polynucleotide of the second library of constructs by removing all, essentially all, or a portion of the inserted artificial transposon from the second library of constructs through restriction endonuclease digestion of the at least two restriction endonuclease recognition sites leaving at least one substitution, deletion, or insertion of at least one nucleotide in the polynucleotide; self-ligating the restriction endonuclease digested fragments; and selecting the insertion library using the third selection marker in a third host cell; and
(d) isolating at least one mutant of the polynucleotide from the insertion library, wherein the isolated mutant comprises at least one substitution, deletion, or insertion of at least one nucleotide in the polynucleotide.

The present invention also relates to methods of producing at least one polynucleotide encoding at least one variant of a parent polypeptide, the method comprising the steps of:
(a) providing a nucleic acid construct comprising a polynucleotide encoding the parent polypeptide, into which polynucleotide has been inserted a heterologous polynucleotide fragment, wherein said fragment comprises at least two restriction endonuclease recognition sites;
(b) restricting the nucleic acid construct with at least two corresponding restriction endonucleases, if necessary in separate individual steps of restricting, PCR-polishing, and ligating, wherein all or essentially all of the inserted heterologous fragment is excised from the construct and at least one nucleotide triplet is deleted, inserted, or substituted in the encoding polynucleotide in the process, whereby at least one polynucleotide encoding at least one variant of the parent polypeptide is produced.

The present invention also relates to polynucleotide constructs comprising a transposon, said transposon comprising one or more outside cutter restriction endonuclease recognition sites.

Disclosed are cells comprising in its genome an integrated heterologous polynucleotide fragment, said fragment comprising one or more outside cutter restriction endonuclease recognition sites.

Disclosed are isolated mutant polynucleotides obtained by such methods; nucleic acid constructs, expression vectors, and host cells comprising such mutant polynucleotides; and methods for producing artificial variants of a polypeptide encoded by such mutant polynucleotides.

### Brief Description of the Figures

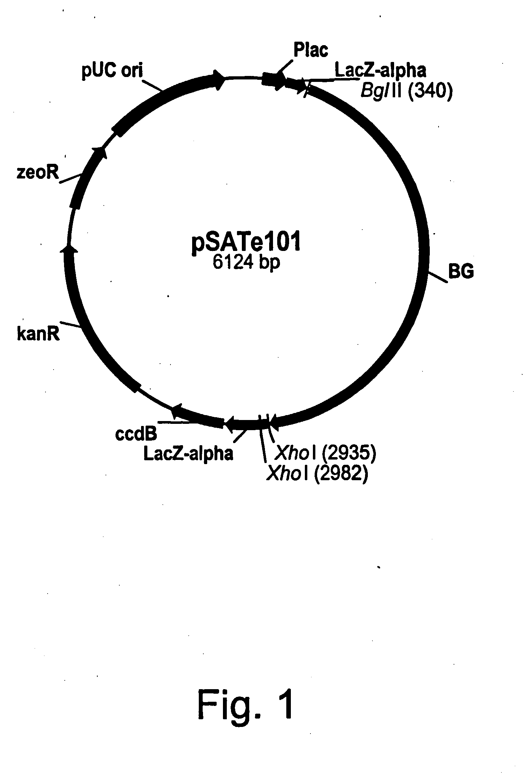

Figure 1 shows a restriction map of pSATe101.

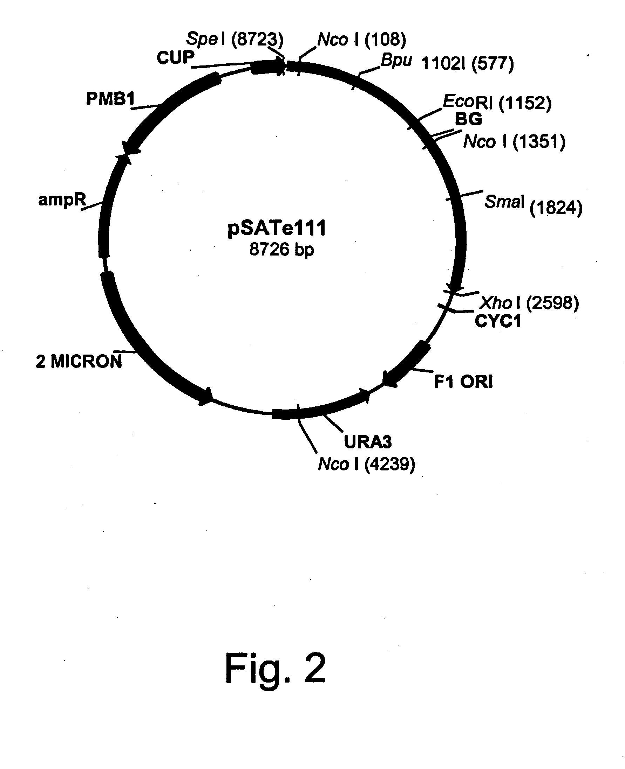

Figure 2 shows a restriction map of pSATe111.

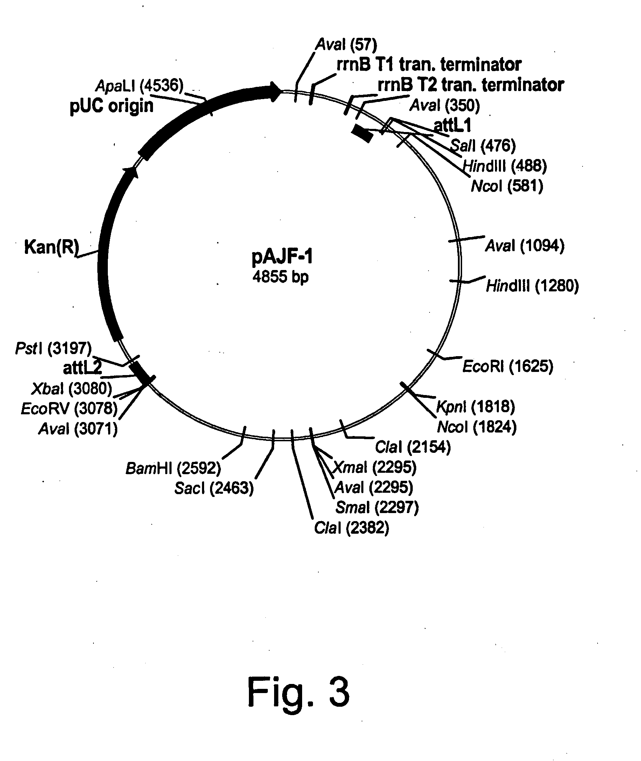

Figure 3 shows a restriction map of pAJF-1.

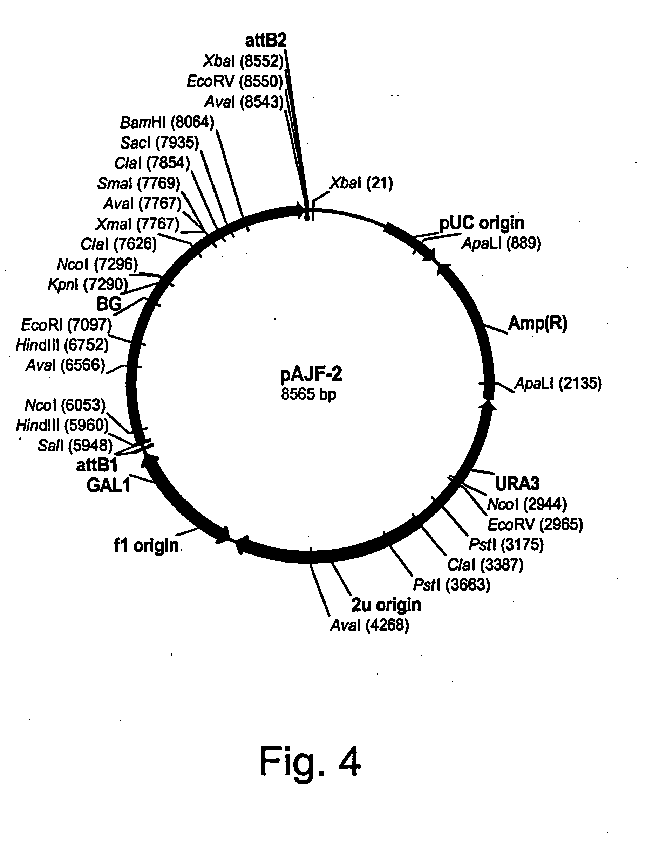

Figure 4 shows a restriction map of pAJF-2.

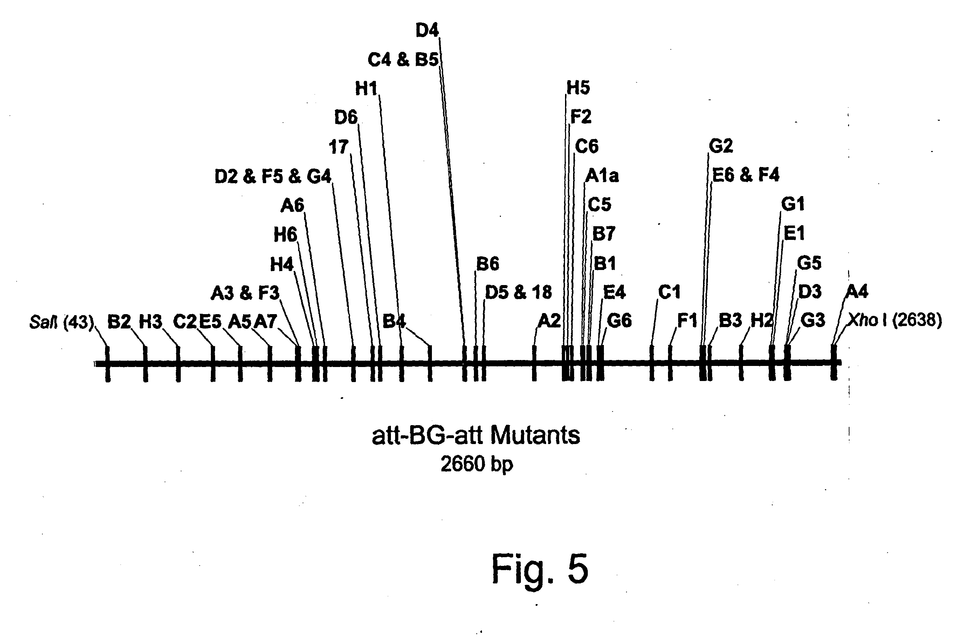

Figure 5 shows the distribution of transposon insertions of an *Aspergillus oryzae* beta-glucosidase gene based on the sequences of 50 clones.

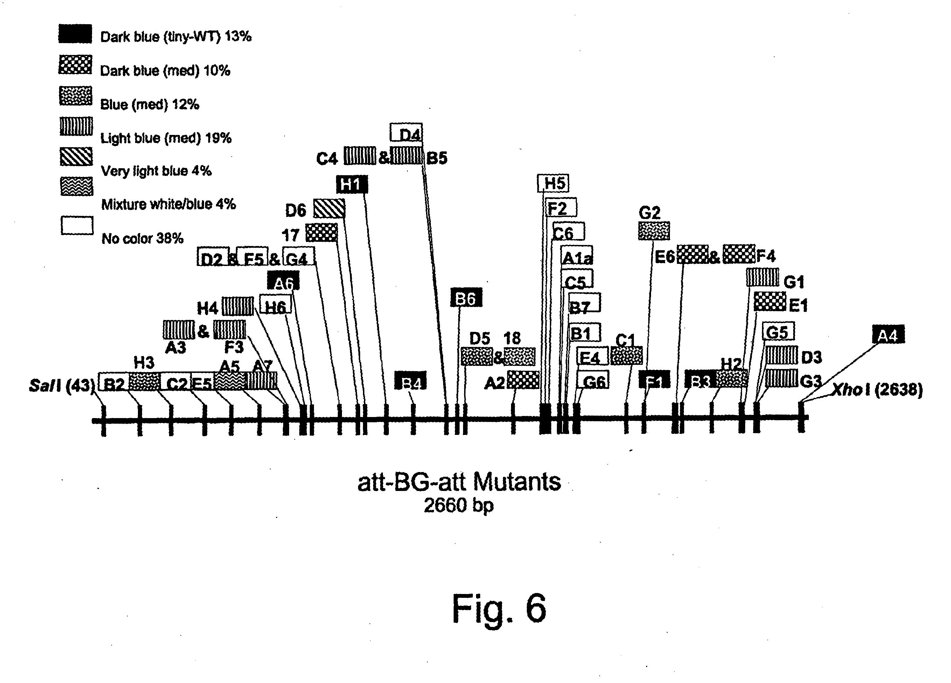

Figure 6 shows phenotype distribution based on transposon insertion position of the *Aspergillus oryzae* beta-glucosidase gene. Each box covering the clone numbers denotes a specific phenotype observed for that clone using a X-glc colorimetric plate assay for beta-glucosidase activity.

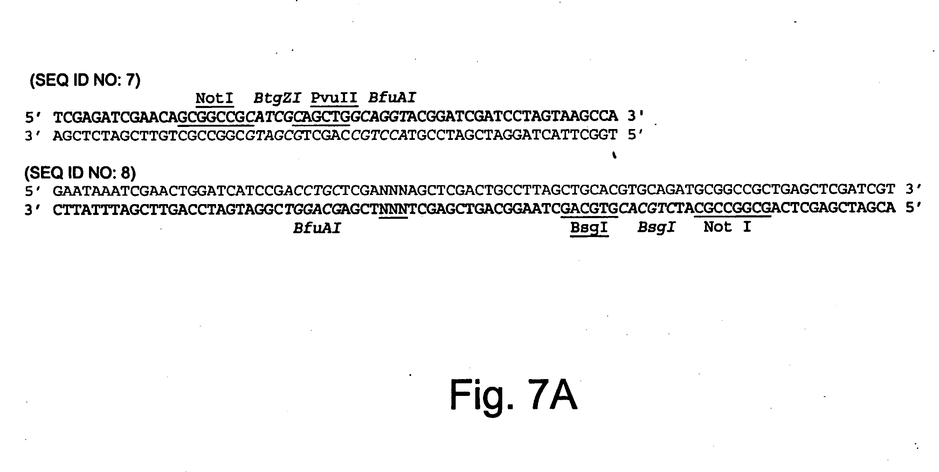

Figure 7A shows two oligonucleotide primers (SEQ ID NO: 7 and SEQ ID NO: 8) designed to PCR-amplify a DNA-fragment suitable to be cloned into the flanking *Not* I-sites of a transposon already inserted in a gene of interest, using the transposon shown in SEQ ID NO: 9 as PCR template. The complementary primer sequences are shown in grey typeface. The primers and consequently also the DNA-fragment comprise a number of restriction endonuclease enzyme recognition sites that are indicated as underlined and/or italicized nucleotides in the sequences, the corresponding enzymes are noted above and below the sequences. In addition, the fragment comprises a random or partially random codon triplet 'NNN'.

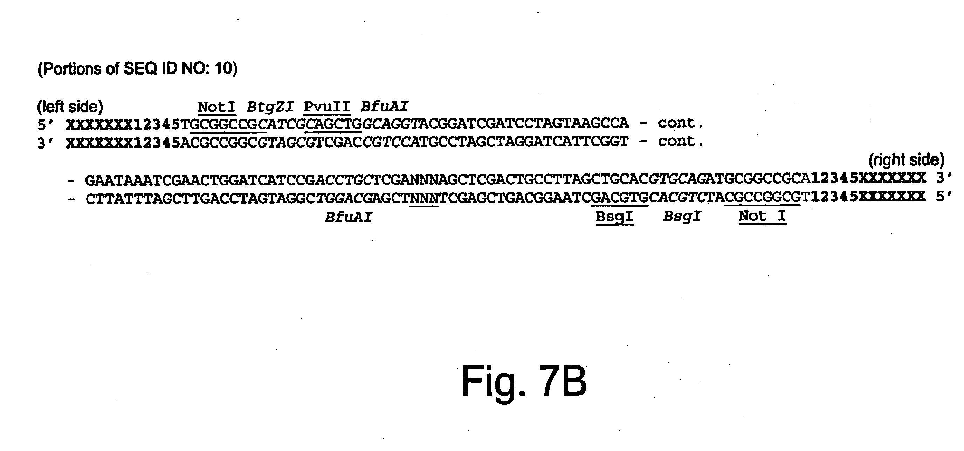

Figure 7B shows ends of the the PCR-fragment after it has been cloned into the transposon in the gene of interest, thus replacing the transposon. The nucleotides in bold typeface, the X'es, and nucleotides 1 through 5, are part of the gene of interest, whereas the normal font nucleotides represent heterologous DNA which has been inserted into the gene. The nucleotides marked 1 through 5 serve to illustrate the target site in the gene of interest where the random or partially random codon triplet 'NNN' will finally be located in the resulting polynucleotide sequence. It is shown that the target site is duplicated by the insertion of the transposon. The full sequence of the transposon with the PCR-fragment cloned into the *Not* I sites is shown in SEQ ID NO: 10.

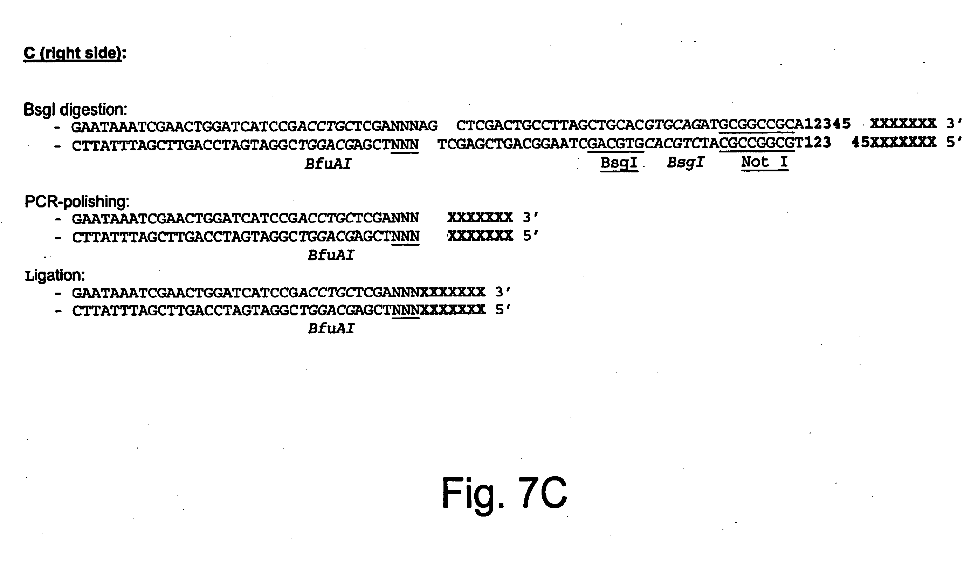

Figure 7C shows how the DNA-fragment has been designed, so that the restriction in C with the outside cutter enzyme *Bsg* I, followed by a PCR-polishing to remove any nucleotide overhangs in the resulting fragments, will bring the right-hand side of the random or partially random triple codon 'NNN' into position directly adjacent to the nucleotides of the gene of interest (shown in bold typeface) after a ligation step.

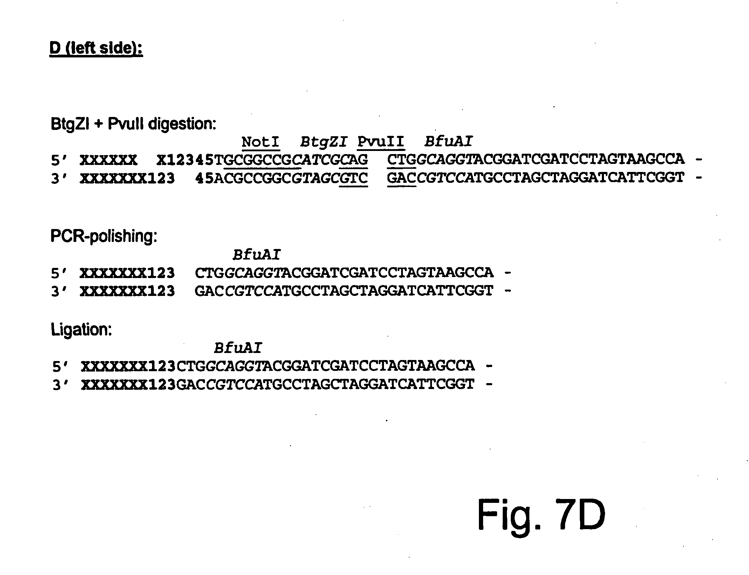

Figure 7D shows how the DNA-fragment has been designed, so that restriction with the outside cutter enzyme *Btg* ZI in combination with the enzyme *Pvu* II, followed by a PCR-polishing filling in of the overhanging nucleotides in the resulting fragments, will bring the DNA-fragment into a suitable position directly adjacent to the nucleotides '1' and '2' of the target site in the gene of interest (in bold typeface) after a ligation step.

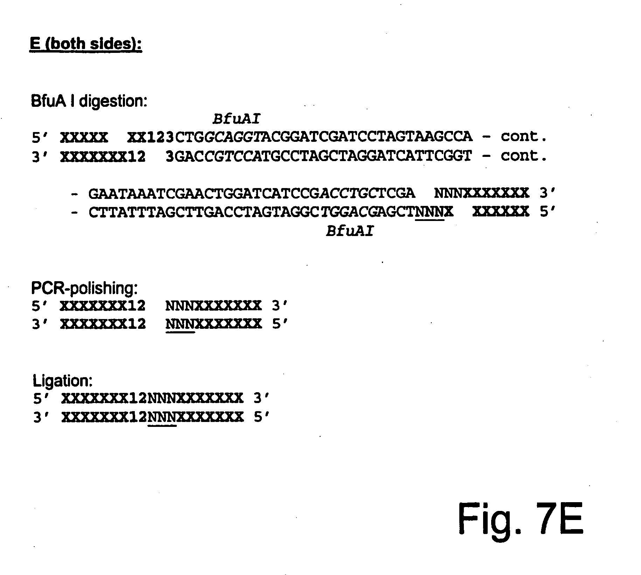

Figure 7E shows the final restriction with the outside cutter enzyme *Bfu* AI, whereby the entire remaining inserted heterologous DNA-fragment is removed from the gene of interest, leaving behind only an overhang of the random or partially random triple codon 'NNN', which after a subsequent PCR-polishing and a ligation step produces a resulting polynucleotide, wherein the nucleotide triplet consisting of nucleotides **'3', '4',** and **'5'** of the target polynucleotide has been replaced with the random or partially random triplet codon denoted by 'NNN'.

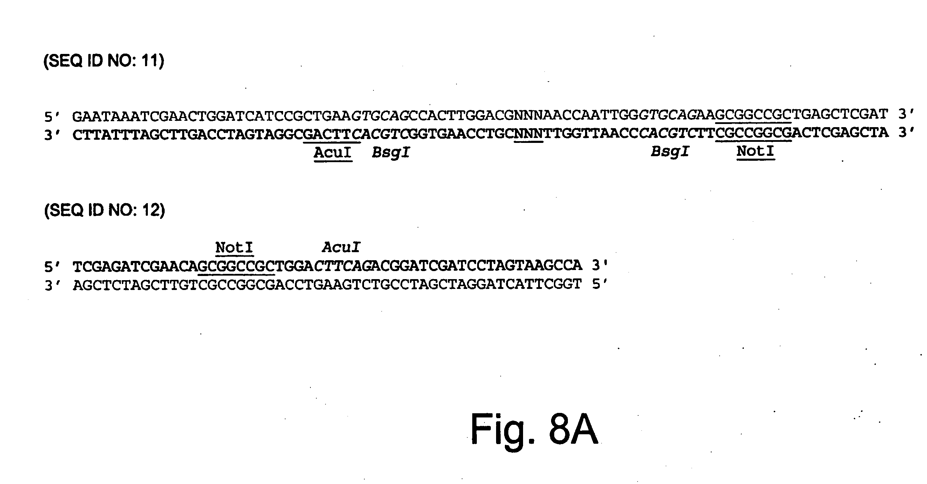

Figure 8A shows two oligonucleotide primers (SEQ ID NO: 11 and SEQ ID NO: 12) designed to PCR-amplify a DNA-fragment suitable to be cloned into the flanking *Not* I-sites of a transposon already inserted in a gene of interest, using the transposon shown in SEQ ID NO: 9 as PCR template. The complementary primer sequences are shown in grey typeface. The primers and consequently also the DNA-fragment comprise a number of restriction endonuclease enzyme recognition sites that are indicated as underlined and/or italicized nucleotides in the sequences, the corresponding enzymes are noted above and below the sequences. In addition, the fragment comprises a random or partially random codon triplet 'NNN'.

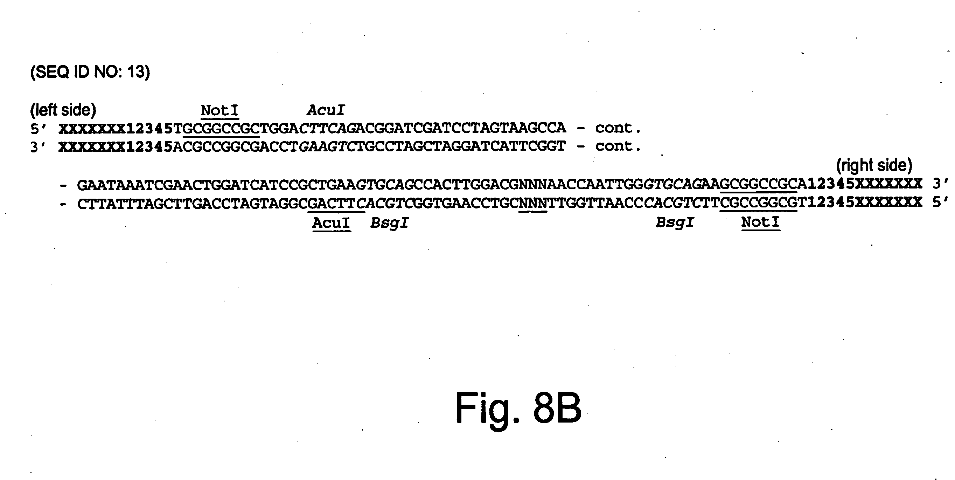

Figure 8B shows the ends of the PCR-fragment after it has been cloned into the transposon in the gene of interest, thus replacing the transposon. The nucleotides in bold typeface, the X'es, and nucleotides 1 through 5, are part of the gene of interest, whereas the normal font nucleotides represent heterologous DNA which has been inserted into the gene. The nucleotides marked 1 through 5 serve to illustrate the target site in the gene of interest where the random or partially random codon triplet 'NNN' will finally be located in the resulting polynucleotide sequence. It is shown that the target site is duplicated by the insertion of the transposon. The full sequence of the transposon with the PCR-fragment cloned into the *Not* I sites is shown in SEQ ID NO: 13.

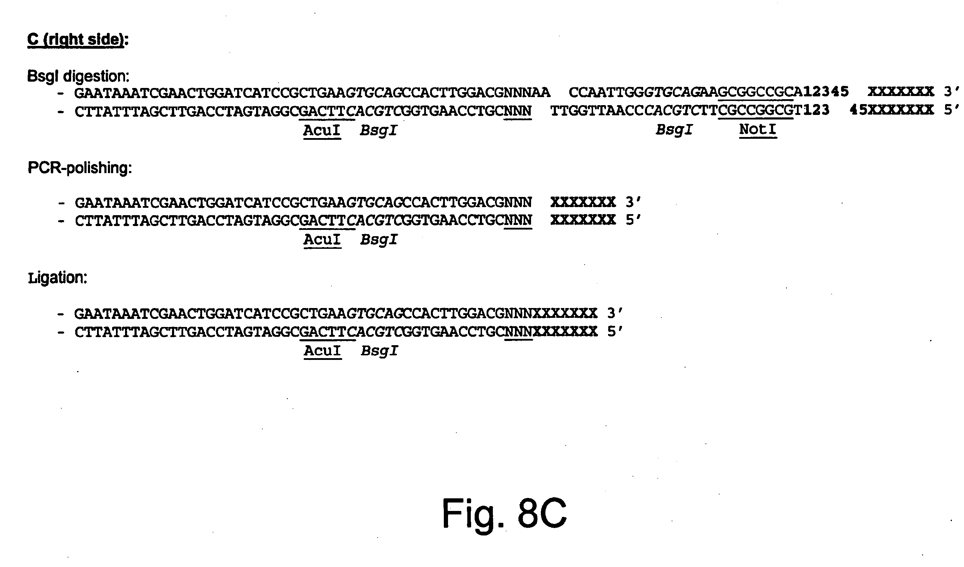

Figure 8C shows how the DNA-fragment of has been designed, so that the restriction in C with the outside cutter enzyme *Bsg* I, followed by a PCR-polishing to remove any nucleotide overhangs in the resulting fragments, will bring the right-hand side of the random or partially random triple codon 'NNN' into position directly adjacent to the nucleotides of the gene of interest (shown in bold typeface) after a ligation step.

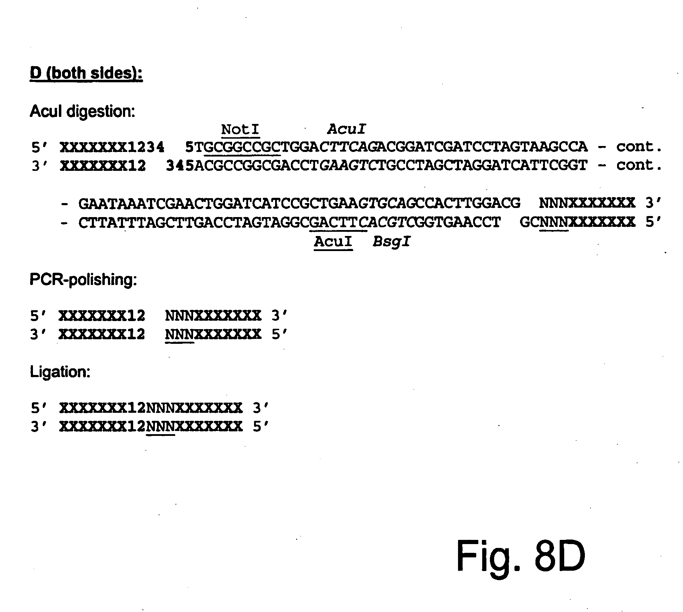

Figure 8D shows the final restriction with the outside cutter enzyme *Acu* I, whereby the entire remaining inserted heterologous DNA-fragment is removed from the gene of interest, leaving behind only an overhang of the random or partially random triple codon 'NNN', which after a subsequent PCR-polishing and a ligation step produces a resulting polynucleotide, wherein the nucleotide triplet consisting of nucleotide **'3', '4',** and **'5'** of the target polynucleotide has been replaced with the random or partially random triplet codon denoted by 'NNN'.

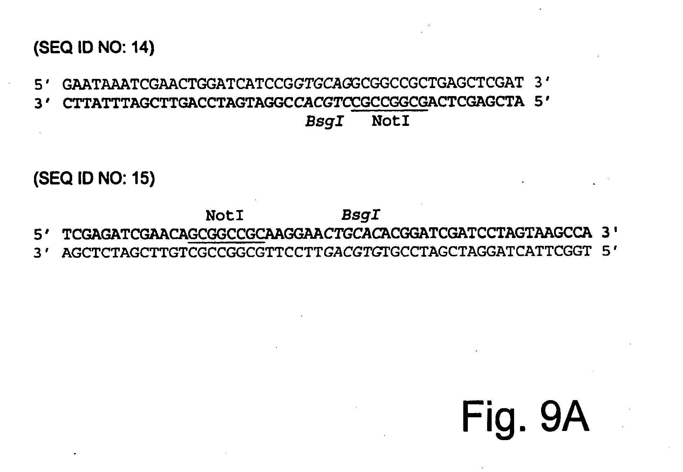

Figure 9A shows two oligonucleotide primers (SEQ ID NO: 14 and SEQ ID NO: 15) designed to PCR-amplify a DNA-fragment suitable to be cloned into the flanking *Not* I-sites of a transposon already inserted in a gene of interest, using the transposon shown in SEQ ID NO: 9 as PCR template. The complementary primer sequences are shown in grey typeface. The primers and consequently also the DNA-fragment comprise a number of restriction endonuclease enzyme recognition sites that are indicated as underlined and/or italicized nucleotides in the sequences, the corresponding enzymes are noted above and below the sequences.

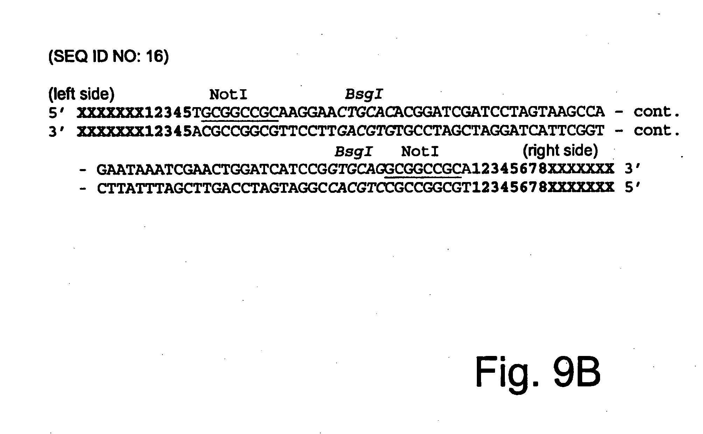

Figure 9B shows the ends of the PCR-fragment after it has been cloned into the transposon in the gene of interest, thus replacing the transposon. The nucleotides in bold typeface, the X'es, and nucleotides 1 through 5 on the left side, and 1 through 8 on the right side, are part of the gene of interest, whereas the normal font nucleotides represent heterologous DNA which has been inserted into the gene. The nucleotides marked 1 through 5 on the left side, and 1 through 7 on the right side, serve to illustrate the target site in the gene of interest where the deleted codon triplet will finally be "located" in the resulting polynucleotide sequence. It is shown that the target site is duplicated by the insertion of the transposon. The full sequence of the transposon with the PCR-fragment cloned into the *Not* I sites is shown in SEQ ID NO: 16.

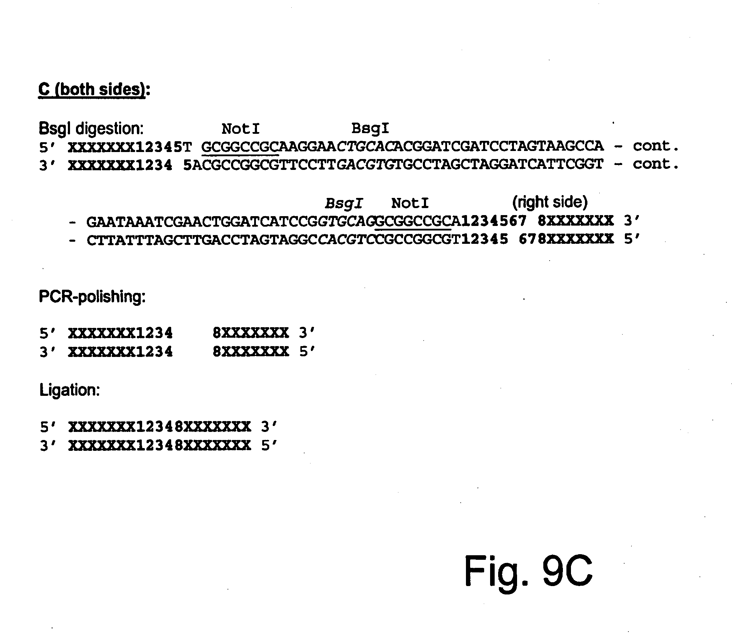

Figure 9C shows restriction with the outside cutter enzyme *Acu* I, whereby the entire remaining inserted heterologous DNA-fragment is removed from the gene of interest, leaving behind only an overhang of the deleted codon triplet, which after a subsequent PCR-polishing and a ligation step produces a resulting polynucleotide, wherein the nucleotide triplet consisting of nucleotides **'5', '6',** and **'7'** in the target polynucleotide has been deleted.

### Definitions

**Inside cutter:** The term "inside cutter" or "inside cutting endonuclease" is defined herein as a restriction endonuclease which digests a DNA sequence inside the actual recognition sequence or site. By far the majority of restriction endonucleases belong to this group. Indeed a very large number of these enzymes are known, and have been known for decades, *e.g*. *Eco* RI or *Bam* HI.

**Outside cutter:** The term "outside cutter" or "outside cutting endonudease" is defined herein as a restriction endonuclease which digests a DNA sequence outside the actual recognition sequence or site. These endonucleases, which are subclasses of Type II enzymes (Szybalski et al., 1991, Gene 100: 13-26), are commercially available from a number of vendors and listed in REBASE. Non-limiting examples of outside cutters are *Aar* I, *Ace* III, *Alf* I, *Alo* I, *Bae* I, *Bbr* 7I, *Bbv* I, *Bbv* II, *Bcc* I, *Bce* 831, *Bce* AI, *Bce* fl, *Bcg* I, Bcl *V*I, *Bfl* I, *Bin* I, *Bpl* I, *Bsa* XI, *Bsa* XI, *Bsc* AI, *Bse* MII, *Bse* RI, *Bsg* I, *BsI* FI, *Bsm* I, *Bsm* AI, *Bsm F*I, Bsp 24I, *Bsp* CNI, *Bsp* MI, *Bsr* I, Bsr DI, *Bst* F5I, *Btg* ZI, *Bts* I, *Cha* I, *Cje* I, *Cje* PI, *Csp* CI, *Cst* MI, *and Eci* I.

**PCR polishing:** The term "PCR polishing" refers to *in vitro* methods of blunt-ending nucleotide overhangs in a polynucleotide fragment after restriction by an endonuclease. Many restriction endonucleases leave behind either a 5' or 3' nucleotide overhang, the so-called "sticky ends", and if two fragments have incompatible overhangs then they cannot be ligated together.

**Isolated polynucleotides:** The term "isolated polynucleotide" or "isolated mutant polynucleotide" as used herein refers to a polynucleotide which is at least 20% pure, preferably at least 40% pure, more preferably at least 60% pure, even more preferably at least 80% pure, most preferably at least 90% pure, and even most preferably at least 95% pure, as determined by agarose electrophoresis.

**Substantially pure polynucleotides:** The term "substantially pure polynucleotide" or "substantially pure mutant polynucleotide" as used herein refers to a polynucleotide preparation free of other extraneous or unwanted nucleotides and in a form suitable for use within genetically engineered production systems. Thus, such substantially pure polynucleotides contain at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polynucleotide material with which it is natively or recombinantly associated. A substantially pure polynucleotide may, however, include naturally occurring 5' and 3' untranslated regions, such as promoters and terminators. It is preferred that the substantially pure polynucleotide is at least 90% pure, preferably at least 92% pure, more preferably at least 94% pure, more preferably at least 95% pure, more preferably at least 96% pure, more preferably at least 97% pure, even more preferably at least 98% pure, most preferably at least 99%, and even most preferably at least 99.5% pure by weight. The polynucleotides of the present invention are preferably in a substantially pure form. In particular, it is preferred that the polynucleotides disclosed herein are in "essentially pure form", *i.e.,* that the polynucleotide preparation is essentially free of other polynucleotide material with which it is natively or recombinantly associated. Herein, the term "substantially pure polynucleotide" is synonymous with the terms "isolated polynucteotid-e" and "polynucleotide in isolated form." The polynucleotides may be of genomic, cDNA, RNA, semisynthetic, synthetic origin, or any combinations thereof.

**cDNA:** The term "cDNA" is defined herein as a DNA molecule which can be prepared by reverse transcription from a mature, spliced, mRNA molecule obtained from a eukaryotic cell. cDNA lacks intron sequences that are usually present in the corresponding genomic DNA. The initial, primary RNA transcript is a precursor to mRNA which is processed through a series of steps before appearing as mature spliced mRNA. These steps include the removal of intron sequences by a process called splicing. cDNA derived from mRNA lacks, therefore, any intron sequences.

**Nucleic acid construct:** The term "nucleic acid construct" or simply "construct" as used herein refers to a nucleic acid molecule, either single- or double-stranded, which is isolated from a naturally occurring gene or which is modified to contain segments of nucleic acids in a manner that would not otherwise exist in nature. The term nucleic acid construct is synonymous with the term "expression cassette" when the nucleic acid construct contains the control sequences required for expression of a coding sequence of the present invention.

**Control sequence:** The term "control sequences" is defined herein to include all components, which are necessary or advantageous for the expression of a polynucleotide encoding an artificial variant of a polypeptide. Each control sequence may be native or foreign to the nucleotide sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

**Operably linked:** The term "operably linked" denotes herein a configuration in which a control sequence is placed at an appropriate position relative to the coding sequence of the polynucleotide sequence such that the control sequence directs the expression of the coding sequence of a polypeptide.

**Coding sequence:** When used herein the term "coding sequence" means a nucleotide sequence, which directly specifies the amino acid sequence of its protein product. The boundaries of the coding sequence are generally determined by an open reading frame, which usually begins with the ATG start codon or alternative start codons "such as GTG and TTG. The coding sequence may a DNA, cDNA, or recombinant nucleotide sequence.

**Expression:** The term "expression" includes any step involved in the production of the polypeptide including, but not limited to, transcription, post-transcriptional modification, translation, post-translational modification, and secretion.

**Expression vector:** The term "expression vector" is defined herein as a linear or circular DNA molecule that comprises a polynucleotide encoding a polypeptide, and which is operably linked to additional nucleotides that provide for its expression.

**Host cell:** The term "host cell", as used herein, includes any cell type which is susceptible to transformation, transfection, transduction, and the like with a nucleic acid construct or expression vector comprising a polynucleotide.

**Modification:** The term "modification" or "modified polynucleotide" means herein any chemical modification as well as genetic manipulation of the DNA encoding that polypeptide. The modification can be substitutions, deletions and/or insertions of one or more amino acids as well as replacements of one or more amino acid side chains.

**Parent polypeptide:** The term "parent polypeptide" as used herein means a polypeptide to which modifications, *e.g*., substitution(s), insertion(s), deletion(s), and/or truncation(s), are made to produce artificial variants. This term also refers to the polypeptide with which a variant is compared and aligned. The parent may be a naturally occurring (wild type) polypeptide, or it may even be a variant thereof, prepared by any suitable means. For instance, the parent polypeptide may be a variant of a naturally occurring polypeptide which has been modified or altered in the amino acid sequence. A parent polypeptide may also be an allelic variant which is a polypeptide encoded by any of two or more alternative forms of a gene occupying the same chromosomal locus.

**Artificial variant:** When used herein, the term "artificial variant" means a polypeptide produced by an organism expressing a modified nucleotide sequence, where the modified nucleotide sequence is obtained through human intervention by modification of the nucleotide sequence.

**Transposon and transposase:** The term "transposon" is defined herein as a region of nucleic acid that is capable of moving from one position to another within DNA where this movement is catalyzed by a transposase. Transposons are also known as "transposable elements".

**Artificial transposon:** When used herein, the term "artificial transposon" means a modified transposon obtained through human intervention by modification of the nucleotide sequence.

**Transposase:** The term "transposase" means a protein that catalyses the steps, *i.e.,* breakage and joining, of a transposition reaction.

***In vitro* transposition:** The term "*in vitro* transposition" is defined herein as a biochemical reaction that is initiated outside the cell that catalyzes the movement of a transposable element from one site into a different site within the same or a different DNA molecule.

***In vivo* transposition:** The term "*in vivo* transposition" means a biochemical reaction that takes place within the cell that catalyzes the mobilization of a transposon from one site to another site within the genome of the host.

**Recombinase:** The term "recombinase" is defined herein as a ubiquitous class of enzymes which catalyze DNA strand recombination in bacteria, yeast, *Drosophila,* immunoglobulin and T cell receptor gene rearrangement, and other systems. Site-specific recombinases include, but are not limited to, bacteriophage P1 Cre recombinase, yeast FLP recombinase, Inti integrase, bacteriophage lambda, phi 80, P22, P2, 186, and P4 recombinase, Tn3 resolvase, the Hin recombinase, the Cin recombinase, *E. coli xerC* and *xerD* recombinases, *Bacillus thuringiensis* recombinase, Tpnl, the beta-lactamase transposons, and the immunoglobulin recombinases.

**Recombination:** The term "recombination" is defined herein as a process wherein nucleic acids associate with each other in regions of homology, leading to interstrand DNA exchange between those sequences. For purposes of the present invention, homologous recombination is determined according to the procedures summarized by Paques and Haber, 1999, Microbiology and Molecular Biology Reviews 63: 349-404. "Homologous recombination" is defined herein as recombination in which no changes in the nucleotide sequences occur within the regions of homology relative to the input nucleotide sequences. For perfect homologous recombination, the regions should contain a sufficient number of nucleic acids, such as 15 to 1,500 base pairs, preferably 100 to 1,500 base pairs, more preferably 400 to 1,500 base pairs, and most preferably 800 to 1,500 base pairs, which are highly homologous with the corresponding nucleic acid sequence to enhance the probability of homologous recombination.

**Improved property:** The term "improved property" is defined herein as a characteristic associated with a mutant polynucleotide which is improved compared to the parent polynucleotide or a variant polypeptide encoded by a mutant polynucleotide which is improved compared to the parent polypeptide. Such improved properties include, but are not limited to, altered control sequence function, altered temperature-dependent activity profile, thermostability, pH activity, pH stability, substrate specificity, product specificity, and chemical stability.

**Altered control sequence function:** The term "altered control sequence function" is defined herein as an alteration of the endogenous function of a control sequence. This may include, but is not limited to, alterations which affect the level of transcription, the stability of the messenger RNA transcribed, the degree or type of messenger RNA processing, the level of secretion, the localization of the controlled protein, or proteolytic processing of the controlled protein.

**Improved thermal activity:** The term "improved thermal activity" is defined herein as an alteration of the temperature-dependent activity profile of a variant enzyme at a specific temperature relative to the temperature-dependent activity profile of the parent enzyme. The thermal activity value provides a measure of the enzyme's efficiency in performing catalysis of a reaction over a range of temperatures. An enzyme has a specific temperature range wherein the protein is stable and retains its enzymatic activity, but becomes less stable and thus less active with increasing temperature. Furthermore, the initial rate of a reaction catalyzed by an enzyme can be accelerated by an increase in temperature which is measured by determining thermal activity of a variant. A more thermoactive variant will lead to an increase in the rate of catalysis decreasing the time required and/or decreasing the enzyme concentration required for catalysis. Alternatively, a variant with a reduced thermal activity will catalyze a reaction at a temperature lower than the temperature optimum of the parent enzyme defined by the temperature-dependent activity profile of the parent.

**Improved thermostability:** The term "improved thermostability" is defined herein as a variant enzyme displaying retention of enzymatic activity after a period of incubation at elevated temperature relative to the parent enzyme. Such a variant may or may not display an altered thermal activity profile relative to the parent. For example, a variant may have an improved ability to refold following incubation at elevated temperature relative to the parent.

In a preferred embodiment, the thermal activity of the variant enzyme is at least 1.5-fold, preferably at least 2-fold, more preferably at least 5-fold, most preferably at least 7-fold, and even most preferably at least 20-fold more thermally active than the wild-type variant under specified conditions.

**Improved product specificity:** The term "improved product specificity" is defined herein as a variant enzyme displaying an altered product profile relative to the parent in which the altered product profile improves the performance of the variant in a given application relative to the parent. The term "product profile" is defined herein as the chemical composition of the reaction products produced by enzymatic catalysis.

**Improved chemical stability:** The term "improved chemical stability" is defined herein as a variant enzyme displaying retention of enzymatic activity after a period of incubation in the presence of a chemical or chemicals, either naturally occurring or synthetic, which reduce the enzymatic activity of the parent enzyme. Improved chemical stability may also result in variants better able to catalyze a reaction in the presence of such chemicals.

### Detailed Description of the Invention

In a first aspect, the present invention relates to methods of producing at least one mutant of a polynucleotide, the method comprising the steps of:
(a) isolating a first library of constructs, wherein each construct comprises a first selectable marker, a polynucleotide, an inserted artificial transposon comprising at least two restriction endonuclease recognition sites and a second selectable marker, and a first recombination site flanking the 5' end of the polynucleotide and a second recombination site flanking the 3' end of the polynucleotide, wherein the artificial transposon has inserted at one or more random sites within the constructs, and wherein the first library is selected using the first and second selectable markers in a first host cell;
(b) isolating a second library of constructs by introducing the first library of constructs into a vector comprising a third selectable marker and a first recombination site and a second recombination site to facilitate site-specific recombination of the first recombination site flanking the 5' end of the polynucleotide and the second recombination site flanking the 3' end of the polynucleotide in the first library of constructs with the first recombination site and the second recombination site of the vector and by selecting the second library of constructs using the second and third selectable markers in a second host cell;
(c) isolating an insertion library containing at least one substitution, deletion, or insertion of at least one nucleotide in each polynucleotide of the second library of constructs by removing all, essentially all, or a portion of the inserted artificial transposon from the second library of constructs through restriction endonuclease digestion of the at least two restriction endonuclease recognition sites leaving at least one substitution, deletion, or insertion of at least one nucleotide in the polynucleotide; self-ligating the restriction endonuclease digested fragments; and selecting the insertion library using the third selection marker in a third host cell; and
(d) isolating at least one mutant of the polynucleotide from the insertion library, wherein the isolated mutant comprises at least one substitution, deletion, or insertion of at least one nucleotide in the polynucleotide.

First Library. In the methods of the present invention, a first library of constructs is isolated, wherein each construct comprises a first selectable marker, a polynucleotide, an inserted artificial transposon comprising at least two restriction endonuclease recognition sites and a second selectable marker, and a first recombination site flanking the 5' end of the polynucleotide and a second recombination site flanking the 3' end of the polynucleotide, wherein the artificial transposon has inserted at one or more random sites within the constructs, and wherein the first library is selected using the first and second selectable markers in a suitable host cell.

In a preferred aspect, the polynucleotide of interest is modified so it contains desired restriction sites to facilitate cloning of the polynucleotide into a vector, for example, an entry vector. PCR can be used in conjunction with specific primers to amplify the polynucleotide of interest to incorporate the desired restriction sites. In a preferred aspect, the polynucleotide of interest is blunt-ended using a thermostable, proofreading polymerase for directionally cloning the polynucleotide into a vector for the "first library of constructs", *e.g*:, an entry vector, and transformation of the vector into a suitable host, *e.g., E. coli.*

Any vector can be used in the methods of the present invention for the "first library of constructs", *e.g*., entry vector. The vector preferably comprises a selectable marker to allow for selection of transformants, two recombination sites to allow recombination into another vector for the "second library of constructs", *e.g*., a destination vector, and an origin of replication for propagation in a host organism, *e.g*., *E. coli, Saccharomyces cerevisiae,* or *Bacillus subtilis.* In the case where the vector comprises two recombination sites, upon ligation of the polynucleotide of interest with the vector, the first recombination site flanks the 5' end of the polynucleotide and the second recombination site flanks the 3' end of the polynucleotide. Alternatively, the polynucleotide of interest can be modified to comprise a first recombination site flanking the 5' end of the polynucleotide and a second recombination site flanking the 3' end of the polynucleotide to facilitate site-specific recombination of the polynucleotide with a vector for the "second library of constructs". For example, two att sites flanking the polynucleotide of interest may be incorporated for recombinase-mediated recombination. In a preferred aspect, the flanking sites consist of at least 3 nucleotides, preferably at least 19 nucleotides, more preferably at least 40 nucleotides, and most preferably at least 60 nucleotides.

In a preferred aspect, the pENTR™ Directional TOPO™ Cloning Kits available from Invitrogen, Carlsbad, CA, are used in the methods of the present invention. Examples of vectors that may be employed in the present invention include, but are not limited to, pENTR™/D-TOPO, pENTR™/SD/D-TOPO, pENTR™/TEV/D-TOPO, pENTR™1A, pENTR™2B, pENTR™3C, pENTR™4, and pENTR™11. These vectors are known commercially as entry vectors.

The vector comprising the polynucleotide of interest is then transformed into a suitable host cell. Any host cell may be used in the methods of the present invention such as those host cells described herein for expression of a mutant polynucleotide. A preferable host cell is, but is not limited to, *E. coli, Saccharomyces cerevisiae,* or *Bacillus subtilis.* Transformants containing the vector with an insert in the correct orientation are then selected, and plasmid DNA isolated and analyzed by restriction analysis, PCR, and/or sequencing for the presence and correct orientation of the insert. Selecting the vector with an insert in the correct orientation enables directional subcloning from the vector into another vector, *e.g*., a destination vector.

The vector comprising the polynucleotide of interest is then subjected to insertional mutagenesis in the presence of an artificial transposon and a transposase to insert the artificial transposon at one or more random positions within the polynucleotide. The artificial transposon preferably comprises 5' and 3' conserved tandem inverted repeats which act as recognition sites for a transposase; a selectable marker gene located within the transposon sequence; and at least two restriction endonuclease recognition sites for transposon and selectable marker removal, and for introduction of one or more substitutions, deletions, or insertions, and self-ligation. Transposase recognition sequences are typically conserved tandom repeats that vary in size depending on the transposition system. For example, the TN7 transposon has two terminal 8-nucleotide inverted repeats.

The randomness of insertion of the transposable element into the polynucleotide of interest can be assessed by preparing DNA, *e.g.,* cosmid DNA, and performing DNA sequencing directed from primers at either ends of the transposon.

The transposase can exist in two different forms. The transposase for Tn5 and Ty1 are made up of a single protein, as are most transposases, and is responsible for target site selection as well as the chemical reactions. In contrast, the Tn7 transposase is made up of several proteins. One set of Tn7 proteins is responsible for selecting the target sites and the other set of Tn7 proteins is needed to carry out the chemical steps of the reaction. A variety of transposases are known in the literature. For a discussion of transposase use and function, see Haren et al., 1999, Annu. Rev. Microbiol. 53, 245-281.

In a preferred aspect, subcloning and expression of a transposase gene are performed from transposons such as Tn5, Tn7 or Mu in a suitable host cell.

Any transposon may be used in the methods of the present invention by modifying the transposon to comprise the above components.

Examples of transposons that may be so modified include, but are not limited to, three distinct types: (1) Retrotransposons (Class I) that first transcribe the DNA into RNA and then use reverse transcriptase to make a DNA copy of the RNA to insert in a new location.; (2) Class II transposons consisting only of DNA that moves directly from place to place; and (3) Class III transposons; also known as Miniature Inverted-Repeats Transposable Elements or MITEs.

A transposable element can be obtained from a suitable source using restriction enzymes and the components described above can be inserted into the transposable element so long as the insertion does not disrupt the inverted repeat sequences that are the binding site for the appropriate transposon. Transposons suitable in the present invention include, but are not limited to, those based upon the yeast Ty1 element, those based upon the bacterial transposon Tn7, the EZ::TN, those based on the bacteriophage Mu, those based on the bacterial transposon Tn552, and the mariner transposable element Himar1 (Lampe et al., 1998, Genetics 149: 179-187), AT-2 (Perkin Elmer; Devine et al., 1997, Genome Res. 7: 551-563), GPS-1 (New England Biolabs), and GPS-2 (New England Biolabs). A number of transposons and methods of identifying and isolating transposons are reviewed by Dyson, 1999, Methods Microbiol. 29: 133-167, incorporated herein by reference. Although these specific transposon systems have been developed for use in *in vitro* systems, it is contemplated that many of the transposon systems, currently only available for *in vivo* transposition, may be modified and developed for *in vitro* work. With appropriate development and characterization, these *in vivo* transposon systems will also be suitable for use in the methods of the present invention.

Although any commercially available *in vitro* transposition system can be used as a mutagenizing tool, the Entranceposon M1-Cam^{R} (Finnzymes Oy, Espoo, Finland) and the Mutation Generation System™ (MGS™, Finnzymes Oy, Espoo, Finland) are preferred to generate transposon insertions in the polynucleotide of interest. The Entranceposon M1-Cam^{R} utilizes the bacteriophage Mu transposase to insert an artificial transposon at random positions within a target DNA population (Mizuuchi, 1992, Annual Review of Biochemistry 61: 1011-1051; Haapa et al., 1999, Nucleic Acids Research 27: 2727-2784). The artificial 1.254 kb transposon used in this system contains the following components: 44 bp 5' and 3' conserved tandem inverted repeats which act as recognition sites for the Mu transposase, *Not* I sites located within the inverted repeats that are used for transposon removal and self-ligation, and internal to these repeats is the coding sequence for a chloramphenicol selection marker.

Other kits for *in vitro* transposition that are commercially available include, for example, The Primer Island Transposition Kit, available from Perkin Elmer Applied Biosystems, Branchburg, N.J., based upon the yeast Ty1 element (including the AT2 transposon); The Genome Priming System, available from New England Biolabs, Beverly, Mass., based upon the bacterial transposon Tn7; and the EZ::TN Transposon Insertion Systems, available from Epicentre Technologies, Madison, Wis., based upon the Tn5 bacterial transposable element.

In the methods of the present invention, the first selectable marker may be any marker that is suitable for use in the host cell of choice. A selectable marker is a gene the product of which provides for biocide or viral resistance, resistance to heavy metals, prototrophy to auxotrophs, and the like to permit easy selection of transformed, transfected, transduced, or the like cells.

Examples of bacterial selectable markers are the *dal* genes from *Bacillus subtilis* or *Bacillus licheniformis,* or markers which confer antibiotic resistance such as ampicillin, kanamycin, chloramphenicol, or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

Second Library. A second library of constructs is isolated by introducing the first library of constructs into a vector comprising a third selectable marker and a first recombination site and a second recombination site to facilitate site-specific recombination of the first recombination site flanking the 5' end of the polynucleotide and the second recombination site flanking the 3' end of the polynucleotide in the first library of constructs with the first recombination site and the second recombination site of the vector and by selecting the second library of constructs using the second and third selectable markers in a suitable host cell.

The recombination reaction is performed in the presence of a recombinase and a vector for the "second library of constructs", *e.g*., a destination vector, to transfer the polynucleotides from the first library of constructs into the vector to generate a second library of constructs or expression clones. Site-specific recombination of the first recombination site flanking the 5' end of the polynucleotide and the second recombination site flanking the 3' end of the polynucleotide in the first library of constructs occurs with the first recombination site and the second recombination site of the vector. The second library of constructs is then selected using the second and third selectable markers.

Any recombinase may be used in the methods of the present invention. In a preferred aspect, LR Clonase™ (Invitrogen, Carlsbad, CA) is used as the recombinase in the present invention. LR Clonase™ is an enzyme mix containing bacteriophage lambda recombination proteins Integrase amd Excisionase and the *E. coli*-encoded protein Integration Host Factor.

Any vector for the "second library of constructs" can be used in the methods of the present invention, such as a destination vector. A large selection of Gateway™ destination vectors are available from Invitrogen, Carlsbad, CA. The vector for the "second library of constructs" preferably comprises a promoter for expression in the host of choice, *e.g*., yeast *GAL1* promoter for galactose-inducible expression in *Saccharomyces cerevisiae*; two recombination sites preferably downstream of the promoter for recombinational cloning of the polynucleotide of interest from the vector for the "first library of constructs"; a selectable marker, *e.g*., chloramphenicol resistance gene, located between the two recombination sites; and an origin of replication for plasmid maintenance in the host. The two recombination sites in the vector for the "second library of constructs" will be the same as or highly homologous to the two recombination sites in the vector for the "first library of constructs". The vector may further comprise one or more of the following components: a negative selection marker, *e.g*., ccdB gene, located between the two recombination sites; a polyadenylation sequence for proper termination and processing of the recombinant transcript; an origin for episomal maintenance and high copy replication, *e.g*., a 2µ origin; an auxotrophic marker for selection in yeast, *e.g*., URA3 auxotrophic marker; an origin for high copy replication and maintenance of the plasmid in *E. coli, e.g.,* pUC origin; and a gene for selection in *E. coli, e.g*., ampicillin resistance gene.

Any promoter capable of driving expression of the polynucleotide is suitable for the present invention. Preferred promoters include, but not limited to, CYC1, HIS3, GAL1, GAL10, ADH1, PGK, PHO5, GAPDH, ADC1, TRP1, URA3, LEU2, ENO, TPI (useful for expression in *Saccharomyces*); AOX1 (useful for expression in *Pichia*); and *lac, ara, tet, trp,* IP_{L}, IP_{R}, T7, tac, and *trc* (useful for expression in *Escherichia coli*) as well as the *amy, apr,* and *npr* promoters and various phage promoters useful for expression in *Bacillus.*

Examples of destination vectors particularly useful in the present invention include, but are not limited to, pBAD-DEST49, pET-DEST42, pDEST™14, pDEST™15, pDEST™17, pDEST™24, and pYES2-DEST52.

The recombination reaction between the two recombination sites on the vector for the "first library of constructs" and the two recombination sites on the vector for the "second library of constructs" preferably replaces the selectable marker gene and the negative selectable marker gene, if present, with the polynucleotide of interest comprising recombination sites in the expression clone.

Following the recombination reaction, the reaction mixture is preferably transformed into a suitable host cell to select for expression clones. Any host cell may be used such as those host cells described herein for expression of a mutant polynucleotide.

In a preferred aspect, competent *E. coli* are used to select for expression clones. Any *recA, endA E. coli* strain including *E. coli* TOP10, DH5α, DH10B, or an equivalent strain, may be used for transformation of the reaction mixture. In the case where the vector for the "second library of constructs" contains a ccdB gene for negative selection, *E. coli* strains that contain the F' episome cannot be used.

In the methods of the present invention, the second and third selectable markers may be any marker that is suitable for use in the host cell of choice as long as they are different from each other and the first selectable marker. Selection with the second and third selectable markers eliminates propagation of the first library of constructs in the second library of constructs.

Transposon mutagenesis, as described herein, can be used to create polynucleotide insertions, deletions, or substitutions by selectively removing some or all or more than the inserted transposon. Using natural or artificial transposons containing restriction endonucleases sites, the inserted transposon and/or target polynucleotide can be selectively cleaved to remove some or all or more than the inserted transposon, and then religated to create the desired insertion, deletion, or substitution. The choice of restriction enzyme or enzymes to be used will depend on whether a substitution, a deletion, or an insertion is being introduced. Roberts et al., 2003, Nucleic Acids Research 31: 418-420 describes various types of restriction endonucleases. Restriction endonucleases can be obtained from numerous commercial suppliers.

By applying transposon technology combined with both Type II restriction endonucleases (restriction endonucleases cutting inside their recognition sequence, hereafter referred to as "inside cutters" as defined herein), it is possible to produce a targeted polynucleotide with one or more nucleotide insertions. Insertions occur wherein the transposon comprises two or more Type II restriction endonuclease recognition sites. For insertions, in theory, any number of nucleotides can be inserted depending on the location of restriction endonuclease cleavage sites within the transposon and subsequent ligation of the remaining transposon.

By applying transposon technology combined with Type IIS or Type IIG restriction endonucleases or any other restriction endonuclease that cleaves a polynucleotide outside their recognition sequence (hereafter referred to as "outside cutters" as defined herein), it is possible to produce targeted polynucleotide libraries with one or more nucleotide deletions. Deletions can be generated when two outside cutter recognition sites are positioned within the inserted transposon such that the outside cutters cleave the target polynucleotide. Religation of the resulting cleavage of the resulting polynucleotide containing the target polynucleotide then results in a mutagenized target polynucleotide deleted in one or more nucleotides.

By applying transposon technology combined with outside cutters, it is also possible to produce targeted polynucleotide libraries with one or more substitutions. For substitutions, one or more nucleotides may be substituted with alternate nucleotides to provide a substitution targeted polynucleotide library.

Substitutions can occur where the transposon comprises two or more outside cutter recognition sites; and more preferably at least one of the one or more outside cutter recognition sites are located so that cleavage with at least one corresponding outside cutter restriction endonuclease results in at least one cut in the targeted polynucleotide located outside of the transposon. By addition and ligation of a linker consisting of a number of nucleotides, subject to the number of nucleotides in the targeted polynucleotide that are removed by cleavage of the outside cutters, one or more substitutions result.

Substitutions can also occur where the use of one or more outside cutter restriction endonucleases results in cleavage of the targeted polynucleotide sequence leaving a set number of nucleotides between the cleavage site and one of the two transposon insertion junctions followed by the use of one or more restriction endonucleases which results in the cleavage of the entire transposon minus the number of nucleotides that are between the cleavage site of the outside cutter restriction endonuclease and one of the two transposon junction sites. Religation of the resulting cleavage of the resulting polynucleotide containing the target polynucleotide then results in a mutagenized target polynucleotide substituted in one or more nucleotides.

Insertion Library. In the methods of the present invention, an insertion library containing at least one substitution, deletion, or insertion of at least one nucleotide in each polynucleotide of the second library of constructs is isolated by removing all, essentially all, or a portion of the inserted artificial transposon from the second library of constructs through restriction endonuclease digestion of the at least two restriction endonuclease recognition sites leaving at least one substitution, deletion, or insertion of at least one nucleotide in the polynucleotide; self-ligating the restriction endonuclease digested fragments; and selecting the insertion library using the third selection marker in a suitable host cell.

The choice of restriction enzyme or enzymes to be used in creating the insertion library will depend on whether a substitution, a deletion, or an insertion is being introduced, as described earlier.

For example, in the Entranceposon M1-Cam^{R} System (Finnzymes Oy, Espoo, Finland), the transposon, after insertion, can be removed using the restriction enzyme *Not* I followed by self-ligation of the backbone which results in a 15 bp in-frame insertion. Ten of 15 bps inserted originate from the inverted repeat sequence that flanks the transposon. The other 5 bp are a result of duplication of the target site that occurs upon integration. The five amino acid insert can be translated into three different peptide combinations based on the insertion frame. In one frame three of the five amino acids are alanines, which is a desired outcome for less deleterious changes to the overall structure of a protein.

In the methods of the present invention, the third selectable marker may be any marker that is suitable for use in the host cell of choice as long as it is different from the first and second selectable markers.

Any host cell may be used in the methods of the present invention such as those host cells described herein for expression of a mutant polynucleotide. A preferable host cell is, but is not limited to, *E. coli, Saccharomyces cerevisiae,* or *Bacillus subtilis.*

In a second aspect, the present invention relates to methods of producing at least one polynucleotide encoding at least one variant of a parent polypeptide, the method comprising the steps of:
(a) providing a nucleic acid construct comprising a polynucleotide encoding the parent polypeptide, into which polynucleotide has been inserted a heterologous polynucleotide fragment, wherein said fragment comprises at least two restriction endonuclease recognition sites;
(b) restricting the nucleic acid construct with at least two corresponding restriction endonucleases, if necessary in separate individual steps of restricting, PCR-polishing, and ligating, wherein all or essentially all of the inserted heterologous fragment is excised from the construct and at least one nucleotide triplet is deleted, inserted, or substituted in the encoding polynucleotide in the process, whereby at least one polynucleotide encoding at least one variant of the parent polypeptide is produced.

Codon triplets and diversity. For a medium sized protein of typically 400 amino acids, a full library covering a single amino acid substitution in one position would be relatively small: 400 x 20 = 8,000 polypeptides, which corresponds to 25,600 polynucleotide coding sequences (using 64 codon triplets). To cover the theoretical diversity in all three reading frames would therefore require 76,800 DNA combinations.

When it is considered that some transposons are inserted into their targets randomly and in either orientation, irrespective of the reading frame, and that a random or partially random codon triplet 'NNN' introduced by the transposon can therefore end up in both orientations and in all reading frames, then the theoretical coding diversity of the 'NNN' triplet can be limited to only 22 codons (excluding stop-codons) in the transposon, rather than 64. For example, if the codon for Trp 'TGG' is positioned to be substituted in one orientation of transposon, the other orientation of transposon would result in the codon 'CCA' (Pro) in the opposite orientation.

Consequently, all twenty amino acid substitutions can in this way be coded for by only 22 different codons in a transposon, as shown in Table 1 below. For a medium sized protein of 400 amino acids the theoretical diversity for all three reading frames would therefore be only 26,400 DNA combinations.

**Table 1. The 22 codons represent all 20 amino acids without stop codons and with only two amino acids (Phe, Val) represented twice. The column 'Codon-1' shows the codons (one direction) for amino acids in column 'AA-1' and the codons in column 'Codon-2' are the complement triplets of the codons (opposite direction) in 'Codon-1' and they code for the amino acids in 'AA-2'.**

| AA-1 | Codon 1 | Codon 2 | AA-2 |
|---|---|---|---|
| Trp | TGG | CCA | Pro |
| Met | ATG | CAT | His |
| Asp | GAT | ATC | Ile |
| Asn | AAC | GTT | Val |
| Lys | AAA | TTT | Phe |
| Glu | GAA | TTC | Phe |
| Tyr | TAC | GTA | Val |
| Gln | CAA | TTG | Leu |
| Cys | TGT | ACA | Thr |
| Ala | GCC | GGC | Gly |
| Ser | TCG | CGA | Arg |

The method of the second aspect comprises several steps, the first of which is the insertion of a transposon into a gene of interest, which gene is preferably located on a plasmid, as described earlier, and which may have been modified to remove any unwanted restriction enzyme sites and/or introns. Gene-fragments with an inserted transposon are then isolated and cloned into a vector, as described earlier. The inserted transposon, which is flanked by restriction enzyme sites, is then replaced in the gene of interest by use of the restriction enzyme(s), *e.g*. *Not* I as illustrated in Figure 1.

A DNA fragment is designed and manufactured comprising a random or partially random triplet codon 'NNN' flanked by "outside cutting" restriction enzyme sites that are flanked in turn by restriction enzyme sites compatible to those flanking the transposon. Alternatively, the transposon may be modified to comprise the outside cutter sites prior to its insertion by transposition into the gene of interest.

For the production of a library of polynucleotides encoding polypeptides having one or more amino acid insertions or substitutions, the use of random or partially random codon triplets is advantageous, often denoted 'NNN'. They may consist of a sharply defined ratio of nucleotides in each position. If the composition in one position is 25% A, 25% G, 25% C, and 25% T, the position is said to be random, *i.e.,* the likelihood is the same for any nucleotide to be present there. However, the ratios may also be adjusted to prefer one or more nucleotides in a given position, in which case it is merely partially random.

Accordingly, in a preferred embodiment, the heterologous polynucleotide fragment or the transposon comprises at least one random or partially random codon triplet 'NNN'.

In another preferred embodiment, the at least two restriction endonuclease recognition sites comprise one or more outside cutter restriction endonuclease recognition site, and preferably restriction with the one or more corresponding outside cutter endonuclease results in one or more cut in the polynucleotide outside of the inserted heterologous polynucleotide fragment.

Another preferred embodiment relates to the method of the second aspect, wherein the at least two restriction endonuclease recognition sites comprise two or more different outside cutter restriction endonuclease recognition sites.

The DNA fragment and a plasmid comprising the gene with the inserted transposon are digested with the compatible restriction enzymes and the DNA fragment is cloned into the gene to replace the transposon.

The outside-cutting sites flanking the inserted DNA-fragment are then restricted with the appropriate outside cutter, if necessary the restricted DNA ends are blunt-ended or filled-in, *e.g*., by PCR polishing, to enable the subsequent ligation (see Figure 7).

Finally the inserted DNA-fragment is excised from the construct by another outside cutter and the construct is ligated, if necessary after the fragments have been blunt-ended or filled-in, so that the three random or partially random base pairs substitute three base pairs of the coding sequence and nothing else of the inserted DNA remains in the construct.

In the resulting polynucleotide only the random or partially random codon triplet 'NNN' remains of the DNA inserted into the gene. This triplet has been brought into position in the coding sequence of the gene of interest and in the process it has replaced three nucleotides of the coding sequence (see Figure 7).

Naturally, more than one codon triplet may be substituted at one time, and by designing the location of the outside cutter recognition sites properly one or more codon triplet may also be inserted and/or deleted. When deletions are intended, all the inserted heterologous sequence will be excised in the process. To achieve insertions or substitutions essentially all of the inserted heterologous sequence will be excised in the process, but of course the respective heterologous inserting and/or substituting coding triplets will necessarily have to be left behind.

In a preferred embodiment, the heterologous polynucleotide fragment comprises a transposon.

In another preferred embodiment, the construct is a DNA plasmid.

In another preferred embodiment, the heterologous polynucleotide fragment or the transposon comprises a selection marker, preferably an antibiotic resistance marker.

In another preferred embodiment, the heterologous polynucleotide fragment or the transposon comprises a polynucleotide having the sequence shown in SEQ ID NO: 10.

Also disclosed is a polynucleotide construct comprising a transposon, said transposon comprising one or more outside cutter restriction endonuclease recognition sites.

The nucleic construct may represent a means for carrying out the method of the second aspect. However, it may also represent an intermediary result after step (a) in the method of the second aspect.

Preferably the transposon comprises two or more outside cutter restriction endonuclease recognition sites; preferably the transposon comprises two or more different outside cutter restriction endonuclease recognition sites; and more preferably at least one of the one or more outside cutter restriction endonuclease recognition site is located so that restriction with at least one corresponding outside cutter restriction endonuclease results in at least one cut in the polynucleotide construct outside of the transposon.

Also disclosed is a cell comprising in its genome an integrated heterologous polynucleotide fragment, said fragment comprising one or more outside cutter restriction endonuclease recognition sites.

The cell may also represent a means for carrying out the method of the first aspect, but also an intermediary result after step (a) in the method of the second aspect.

Preferably, the heterologous polynucleotide fragment comprises a transposon, wherein the one or more outside cutter restriction endonuclease recognition site is comprised in the transposon; preferably the heterologous polynucleotide fragment comprises two or more outside cutter restriction endonuclease recognition sites; and more preferably the heterologous polynucleotide fragment comprises two or more different outside cutter restriction endonuclease recognition sites.

Aternatively at least one of the one or more outside cutter restriction endonuclease recognition site is located so that restriction with at least one corresponding outside cutter restriction endonuclease results in at least one cut in the genome of the cell outside of the integrated heterologous polynucleotide fragment.

### Polynucleotides

The polynucleotide of interest can be any polynucleotide and can be obtained from any prokaryotic, eukaryotic, or other source. For purposes of the present invention, the term "obtained from" as used herein in connection with a given source shall mean that the polynucleotide is native to the source or is from a source into which the polynucleotide had been inserted. In a preferred aspect, the polynucleotide of interest encodes a polypeptide that is secreted extracellularly.

Techniques used to isolate or clone a polynucleotide of interest are known in the art and include isolation from genomic DNA, preparation from cDNA, or a combination thereof. The cloning of the polynucleotide from such genomic DNA can be effected, *e.g*., by using the well known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligated activated transcription (LAT), and nucleotide sequence-based amplification (NASBA) may be used. Standard recombinant DNA and molecular cloning techniques used herein are well known in the art and are described by Sambrook, J., Fritsch, E. F. and Maniatis, T., Molecular Cloning: A Laboratory Manual, Second Edition, Cold Spring Harbor Laboratory Press, Cold Spring Harbor, N.Y. (1989); and by Silhavy, T. J., Bennan, M. L. and Enquist, L. W., Experiments with Gene Fusions, Cold Spring Harbor Laboratory Cold Press Spring Harbor, N.Y. (1984); and by Ausubel, F. M. et al., Current Protocols in Molecular Biology, published by Greene Publishing Assoc. and Wiley-Interscience (1987). The polynucleotide may be of genomic, cDNA, RNA, semisynthetic, synthetic origin, or any combinations thereof.

The polynucleotide of interest may encode a polypeptide such as an antibody, hormone, enzyme, receptor, reporter, or selectable marker. The polypeptide is preferably secreted extracellularly.

In a preferred aspect, the polypeptide is an oxidoreductase, transferase, hydrolase, lyase, isomerase, or ligase. In a more preferred aspect, the polypeptide is an aminopeptidase, amylase, beta-glucosidase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lactonohydrolase, lipase, lysozyme, mannosidase, mutanase, oxidase, pectinolytic enzyme, peroxidase, phospholipase, phytase, polyphenoloxidase, proteolytic enzyme, ribonuclease, transglutaminase, or xylanase.

A polypeptide can also include fused polypeptides or cleavable fusion polypeptides in which another polypeptide is fused at the N-terminus or the C-terminus of a polypeptide or fragment thereof. A fused polypeptide is produced by fusing another nucleotide sequence (or a portion thereof) encoding another polypeptide to a nucleotide sequence (or a portion thereof) encoding a polypeptide. Techniques for producing fusion polypeptides are known in the art, and include ligating the coding sequences encoding the polypeptides so that they are in frame and that expression of the fused polypeptide is under control of the same promoter(s) and terminator.

The polynucleotide of interest can also be a control sequence such as a leader, polyadenylation sequence, propeptide sequence, promoter, signal peptide sequence, or transcription terminator.

The polynucleotide of interest can also be an origin of replication.

The polynucleotide of interest may be bacterial in origin. For example, the polynucleotide may be obtained from a gram positive bacterium such as a *Bacillus* or *Streptomyces,* or a gram negative bacterium.

In a preferred aspect, the polynucleotide is obtained from *Bacillus alkalophilus, Bacillus amyloliquefaciens, Bacillus brevis, Bacillus circulans, Bacillus coagulans, Bacillus lautus, Bacillus lentus, Bacillus licheniformis, Bacillus megaterium, Bacillus stearothermophilus, Bacillus subtilis, Bacillus thuringiensis, Streptomyces lividans,* or *Streptomyces murinus.* In another preferred aspect, the polynucleotide is obtained from *E. coli* or *Pseudomonas* sp.

The polynucleotide of interest may also be fungal in origin, and preferably from a yeast such as *Candida, Kluyveromyces, Pichia, Saccharomyces, Schizosaccharomyces,* or *Yarrowia*; or preferably from a filamentous fungus such as *Acremonium, Aspergillus, Aureobasidium,* Cryptococcus, *Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* or *Trichoderma.*

In a preferred aspect, the polynucleotide is obtained from *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis.*

In another preferred aspect, the polynucleotide is obtained from *Aspergillus aculeatus, Aspergillus awamori, Aspergillus fumigatus, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Trichoderma harzianum, Trichoderma koningii, Trichoderma longibrachiatum, Trichoderma reesei,* or *Trichoderma viride.*

It will be understood that for the aforementioned species the invention encompasses both the perfect and imperfect states, and other taxonomic equivalents, *e.g*., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

A polynucleotide of interest may be identified and obtained from other sources including microorganisms isolated from nature (*e.g*., soil, composts, water, etc.) using the above-mentioned probes. Techniques for isolating microorganisms from natural habitats are well known in the art. The polynucleotide may then be obtained by similarly screening a genomic or cDNA library of another microorganism. Once a polynucleotide sequence encoding a polypeptide has been detected with the probe(s), the polynucleotide can be isolated or cloned by utilizing techniques which are well known to those of ordinary skill in the art (see, *e.g*., Sambrook *et al.,* 1989, *supra*).

### Isolation of a Mutant of the Polynucleotide

Techniques used to isolate or clone a mutant of a polynucleotide of interest from the insertion library are known in the art and include isolation from genomic DNA, preparation from cDNA, or a combination thereof. The cloning of the polynucleotide from such genomic DNA can be effected, *e.g*., by using the well known polymerase chain reaction (PCR) or antibody screening of expression libraries to detect cloned DNA fragments with shared structural features. See, *e.g*., Innis *et al.,* 1990, *supra.* Other nucleic acid amplification procedures such as ligase chain reaction (LCR), ligated activated transcription (LAT) and nucleotide sequence-based amplification (NASBA) may be used.

### Conventions for Designation of Variants

In the present invention, specific numbering of amino acid residue positions is employed in the protein variants. For example, by aligning the amino acid sequences of known proteins having the same biological function, it is possible to designate an amino acid position number to any amino acid residue in any specific protein.

Multiple alignments of protein sequences may be made, for example, using "ClustalW" (Thompson, J.D., Higgins, D.G. and Gibson, T.J., 1994, CLUSTAL W: Improving the sensitivity of progressive multiple sequence alignment through sequence weighting, positions-specific gap penalties and weight matrix choice, Nucleic Acids Research 22: 4673-4680). Multiple alignments of DNA sequences may be done using the protein alignment as a template, replacing the amino acids with the corresponding codon from the DNA sequence.

Pairwise sequence comparison algorithms in common use are adequate to detect similarities between protein sequences that have not diverged beyond the point of approximately 20-30% sequence identity (Doolittle, 1992, Protein Sci. 1: 191-200; Brenner et al., 1998, Proc. Natl. Acad Sci. USA 95, 6073-6078). However, truly homologous proteins with the same fold and similar biological function have often diverged to the point where traditional sequence-based comparisons fail to detect their relationship (Lindahl and Elofsson, 2000, J. Mol. Biol. 295: 613-615). Greater sensitivity in sequence-based searching can be attained using search programs that utilize probabilistic representations of protein families (profiles) to search databases. For example, the PSI-BLAST program generates profiles through an iterative database search process and is capable of detecting remote homologs (Atschul et al., 1997, Nucleic Acids Res. 25: 3389-3402). Even greater sensitivity can be achieved if the family or superfamily for the protein of interest has one or more representatives in the protein structure databases. Programs such as GenTHREADER (Jones 1999, J. Mol. Biol. 287: 797-815; McGuffin and Jones, 2003, Bioinformatics 19: 874-881) utilize information from a variety of sources (PSI-BLAST, secondary structure prediction, structural alignment profiles, and solvation potentials) as input to a neural network that predicts the structural fold for a query sequence. Similarly, the method of Gough et al., 2000, J. Mol. Biol. 313: 903-919, can be used to align a sequence of unknown structure with the superfamily models present in the SCOP database. These alignments can in turn be used to generate homology models for the protein of interest, and such models can be assessed for accuracy using a variety of tools developed for that purpose.

For proteins of known structure, several tools and resources are available for retrieving and generating structural alignments. For example the SCOP superfamilies of proteins have been structurally aligned, and those alignments are accessible and downloadable. These alignments can be used to predict the structurally and functionally corresponding amino acid residues in proteins within the same structural superfamily. This information, along with information derived from homology modeling and profile searches, can be used to predict which residues to mutate when moving mutations of interest from one protein to a close or remote homolog.

In describing the protein variants of the present invention, the nomenclature described below is adapted for ease of reference. In all cases, the accepted IUPAC single letter or triple letter amino acid abbreviation is employed.

Substitutions. For an amino acid substitution, the following nomenclature is used: Original amino acid, position, substituted amino acid. Accordingly, the substitution of threonine with alanine at position 226 is designated as "Thr226Ala" or "T226A". Multiple mutations are separated by addition marks ("+"), *e.g*., "Gly205Arg + Ser411 Phe" or "G205R + S411 F", representing mutations at positions 205 and 411 substituting glycine (G) with arginine (R), and serine (S) with phenylalanine (F), respectively.

Deletions. For an amino acid deletion, the following nomenclature is used: Original amino acid, position*. Accordingly, the deletion of glycine at position 195 is designated as "ugly195*" or "G195*". Multiple deletions are separated by addition marks ("+"), *e.g*., "Gly195* + Ser411*" or "G195* + S411*".

Insertions. For an amino acid insertion, the following nomenclature is used: Original amino acid, position, original amino acid, new inserted amino acid. Accordingly the insertion of lysine after glycine at position 195 is designated "Gly195GlyLys" or "G195GK".

Multiple modifications. Variants comprising multiple modifications are separated by addition marks ("+"), *e.g*., "Arg170Tyr+Gly195Glu" or "R170Y+G195E" representing modifications at positions 170 and 195 substituting tyrosine and glutamic acid for arginine and glycine, respectively.

The artificial variants may comprise a conservative substitution, deletion, and/or insertion of one or more amino acids that, for example, do not significantly affect the folding and/or activity of the protein; small deletions, typically of one to about 30 amino acids; or small amino- or carboxyl-terminal extensions, such as an amino-terminal methionine residue.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In addition to the 20 standard amino acids, non-standard amino acids (such as 4-hydroxyproline, 6-N-methyl lysine, 2-aminoisobutyric acid, isovaline, and alpha-methyl serine) may be substituted for amino acid residues of a wild-type polypeptide. A limited number of non-conservative amino acids, amino acids that are not encoded by the genetic code, and unnatural amino acids may be substituted for amino acid residues. "Unnatural amino acids" have been modified after protein synthesis, and/or have a chemical structure in their side chain(s) different from that of the standard amino acids. Unnatural amino acids can be chemically synthesized, and preferably, are commercially available, and include pipecolic acid, thiazolidine carboxylic acid, dehydroproline, 3- and 4-methylproline, and 3,3-dimethylproline.

Alternatively, the amino acid changes are of such a nature that the physicochemical properties of the polypeptides are altered. For example, amino acid changes may improve the thermal stability of the polypeptide, alter the substrate specificity, change the pH optimum, and the like. The artificial variants may comprise a substitution, deletion, and/or insertion of one or more essential amino acids in the parent polypeptide. Essential amino acids can be identified according to procedures known in the art, such as site-directed mutagenesis or alanine-scanning mutagenesis (Cunningham and Wells, 1989, Science 244:1081-1085). The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309:59-64. The identities of essential amino acids can also be inferred from analysis of identities with polypeptides which are related to a polypeptide according to the invention.

In a preferred embodiment, a mutant polynucleotide or a variant polypeptide has an improved property compared to the parent polynucleotide or the parent polypeptide, respectively. Such improved properties include, but are not limited to, altered control sequence function, altered temperature-dependent activity profile, thermostability, pH activity, pH stability, substrate specificity, product specificity, and chemical stability.

### Nucleic Acid Constructs

The present invention also relates to nucleic acid constructs comprising an isolated mutant polynucleotide encoding an artificial variant of a parent polypeptide operably linked to one or more control sequences which direct the expression of the coding sequence in a suitable host cell under conditions compatible with the control sequences.

An isolated mutant polynucleotide encoding an artificial variant of the present invention may be manipulated in a variety of ways to provide for expression of the artificial variant Manipulation of the polynucleotide's sequence prior to its insertion into a vector may be desirable or necessary depending on the expression vector. The techniques for modifying polynucleotide sequences utilizing recombinant DNA methods are well known in the art.

The control sequence may be an appropriate promoter sequence, a nucleotide sequence which is recognized by a host cell for expression of a mutant polynucleotide encoding an artificial variant of a polypeptide. The promoter sequence contains transcriptional control sequences which mediate the expression of the polypeptide. The promoter may be any nucleotide sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention, especially in a bacterial host cell, are the promoters obtained from the *E. coli lac* operon, *Streptomyces coelicolor* agarase gene (*dagA*), *Bacillus subtilis* levansucrase gene (*sacB*), *Bacillus licheniformis* alpha-amylase gene (*amyL*), *Bacillus stearothermophilus* maltogenic amylase gene (*amyM*), *Bacillus amyloliquefaciens* alpha-amylase gene (*amyQ*), *Bacillus licheniformis* penicillinase gene (*penP*), *Bacillus subtilis xylA* and *xylB* genes, and prokaryotic beta-lactamase gene (Villa-Kamaroff et al., 1978, Proceedings of the National Academy of Sciences USA 75: 3727-3731), as well as the tac promoter (DeBoer et al., 1983, Proceedings of the National Academy of Sciences USA 80: 21-25). Further promoters are described in "Useful proteins from recombinant bacteria" in Scientific American, 1980, 242: 74-94; and in Sambrook *et al.,* 1989, *supra.*

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase (WO 00/56900), *Fusarium venenatum* Daria (WO 00/56900), *Fusarium venenatum* Quinn (WO 00/56900), *Fusarium oxysporum* trypsin-like protease (WO 96/00787). *Trichoderma reesei* beta-glucosidase, *Trichoderma reesei* cellobiohydrolase I, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase *I, Trichoderma reesei* xylanase II, *Trichoderma reesei* beta-xylosidase, as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionine (CUP1), and *Saccharomyces cerevisiae* 3-phosphoglycerate kinase. Other useful promoters for yeast host cells are described by Romanos et al., 1992, Yeast 8: 423-488.

The control sequence may also be a suitable transcription terminator sequence, a sequence recognized by a host cell to terminate transcription. The terminator sequence is operably linked to the 3' terminus of the nucleotide sequence encoding the artificial variant of a polypeptide. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be a suitable leader sequence, a nontranslated region of an mRNA which is important for translation by the host cell. The leader sequence is operably linked to the 5' terminus of the nucleotide sequence encoding the artificial variant of a polypeptide. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3' terminus of the nucleotide sequence and which, when transcribed, is recognized by the host cell as a signal to add polyadenosine residues to transcribed mRNA. Any polyadenylation sequence which is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

Useful polyadenylation sequences for yeast host cells are described by Guo and Sherman, 1995, Molecular Cellular Biology 15: 5983-5990.

The control sequence may also be a signal peptide coding region that codes for an amino acid sequence linked to the amino terminus of an artificial variant of a polypeptide and directs the encoded polypeptide into the cell's secretory pathway. The 5' end of the coding sequence of the nucleotide sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for bacterial host cells are the signal peptide coding regions obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases (*nprT, nprS, nprM*), and *Bacillus subtilis prsA*. Further signal peptides are described by Simonen and Palva, 1993, Microbiological Reviews 57: 109-137.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, *Humicola insolens* endoglucanase V, and *Humicola lanuginosa* lipase.

Useful signal peptides for yeast host cells are obtained from the genes for *Saccharomyces cerevisiae* alpha-factor and *Saccharomyces cerevisiae* invertase. Other useful signal peptide coding regions are described by Romanos *et al*., 1992, *supra*.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (*aprE*), *Bacillus subtilis* neutral protease (*nprT*), *Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of an artificial variant of a polypeptide, the propeptide region is positioned next to the amino terminus of a polypeptide and the signal peptide region is positioned next to the amino terminus of the propeptide region.

It may, also be desirable to add regulatory sequences which allow the regulation of the expression of the artificial variant of a polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleotide sequence encoding the polypeptide would be operably linked with the regulatory sequence.

### Expression Vectors

The present invention also relates to recombinant expression vectors comprising a mutant polynucleotide encoding an artificial variant of the present invention, a promoter, and transcriptional and translational stop signals. The various nucleotide and control sequences described above may be joined together to produce a recombinant expression vector which may include one or more convenient restriction sites to allow for insertion or substitution of the nucleotide sequence encoding the artificial variant at such sites. Alternatively, the nucleotide sequence may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) which can be conveniently subjected to recombinant DNA procedures and can bring about expression of the nucleotide sequence. The choice of the vector will typically depend on the compatibility of the vector with the host cell into which the vector is to be introduced. The vectors may be linear or closed circular plasmids.

The vector may be an autonomously replicating vector, *i*.*e*., a vector which exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed, transfected, transduced, or the like cells. A selectable marker, as described earlier, is a gene the product of which provides for biocide or viral resistance, resistance to heavy metals, prototrophy to auxotrophs, and the like.

Examples of bacterial selectable markers are the *dal* genes from *Bacillus subtilis* or *Bacillus licheniformis*, or markers which confer antibiotic resistance such as ampicillin, kanamycin, chloramphenicol, or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (omithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

The vectors of the present invention preferably contain an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or nonhomologous recombination. Alternatively, the vector may contain additional nucleotide sequences for directing integration by homologous recombination into the genome of the host cell at a precise location(s) in the chromosome(s). To increase the likelihood of integration at a precise location, the integrational elements should preferably contain a sufficient number of nucleic acids, such as 100 to 10,000 base pairs, preferably 400 to 10,000 base pairs, and most preferably 800 to 10,000 base pairs, which have a high degree of identity with the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication which functions in a cell. The term "origin of replication" or "plasmid replicator" is defined herein as a nucleotide sequence that enables a plasmid or vector to replicate *in vivo*.

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98:61-67; Cullen et al., 1987, Nucleic Acids Research 15: 9163-9175; WO 00/24883). Isolation of the AMA1 gene and construction of plasmids or vectors comprising the gene can be accomplished according to the methods disclosed in WO 00/24883.

More than one copy of a mutant polynucleotide of the present invention may be inserted into the host cell to increase production of the gene product. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the components described above to construct the recombinant expression vectors of the present invention are well known to one skilled in the art (see, e.g., Sambrook *et al*., 1989, *supra*).

### Host Cells

The present invention also relates to recombinant host cells, comprising a mutant polynucleotide sequence encoding an artificial variant, which are advantageously used in the recombinant production of the artificial variant. A vector comprising a mutant polynucleotide of the present invention is introduced into a host cell so that the vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the artificial variant and its source.

The host cell may be a unicellular microorganism, e.g., a prokaryote, or a non-unicellular microorganism, e.g., a eukaryote.

Useful unicellular microorganisms are bacterial cells such as gram positive bacteria including, but not limited to, a *Bacillus* cell, *e.g., Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis,* and *Bacillus thuringiensis;* or a *Streptomyces* cell, *e.g., Streptomyces lividans* and *Streptomyces murinus,* or gram negative bacteria such as *E. coli* and *Pseudomonas* sp. In a preferred aspect, the bacterial host cell is a *Bacillus lentus, Bacillus licheniformis, Bacillus stearothermophilus,* or *Bacillus subtilis* cell. In another preferred aspect, the *Bacillus* cell is an alkalophilic *Bacillus*.

The introduction of a vector into a bacterial host cell may, for instance, be effected by protoplast transformation (see, e.g., Chang and Cohen, 1979, Molecular General Genetics 168: 111-115), using competent cells (see, e.g., Young and Spizizin, 1961, Journal of Bacteriology 81: 823-829, or Dubnau and Davidoff-Abelson, 1971, Journal of Molecular Biology 56: 209-221), electroporation (see, *e.g.,* Shigekawa and Dower, 1988, Biotechniques 6: 742-751), or conjugation (see, e.g., Koehler and Thome, 1987, Journal of Bacteriology 169: 5771-5278).

The host cell may also be a eukaryote, such as a mammalian, insect, plant, or fungal cell.

In a preferred aspect, the host cell is a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth *et al*., 1995, *supra*, page 171) and all mitosporic fungi (Hawksworth *et al*., 1995, *supra*).

In a more preferred aspect, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F.A., Passmore, S.M., and Davenport, R.R., eds, Soc. App. Bacteriol. Symposium Series No. 9, 1980).

In an even more preferred aspect, the yeast host cell is a *Candida, Hansenula, Kluyveromyces, Pichia, Saccharomyces,* Schizosaccharomyces, or *Yarrowia* cell.

In a most preferred aspect, the yeast host cell is a *Saccharomyces carlsbergensis*, *Saccharomyces cerevisiae, Saccharomyces diastaticus, Saccharomyces douglasii, Saccharomyces kluyveri, Saccharomyces norbensis*, or *Saccharomyces oviformis* cell. In another most preferred aspect, the yeast host cell is a *Kluyveromyces lactis* cell. In another most preferred aspect, the yeast host cell is a *Yarrowia lipolytica* cell.

In another more preferred aspect, the fungal host cell is a filamentous fungal cell. "Filamentous fungi" include all filamentous forms of the subdivision Eumycota and Oomycota (as defined by Hawksworth *et al*., 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In an even more preferred aspect, the filamentous fungal host cell is an *Acremonium, Aspergillus, Aureobasidium, Bjerkandera, Ceriporiopsis, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus*, *Thielavia, Tolypocladium, Trametes,* or *Trichoderma* cell.

In a most preferred aspect, the filamentous fungal host cell is an *Aspergillus awamori, Aspergillus fumigatus, Aspergillus foetidus, Aspergillus japonicus, Aspergillus nidulans, Aspergillus niger* or *Aspergillus oryzae* cell. In another most preferred aspect, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another most preferred aspect, the filamentous fungal host cell is a *Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Coprinus cinereus, Coriolus hirsutus, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known per se. Suitable procedures for transformation of *Aspergillus* and *Trichoderma* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

### Methods of Production

The present invention also relates to methods for producing an artificial variant of a parent polypeptide, comprising (a) cultivating a host cell comprising a mutant polynucleotide encoding the variant under conditions conducive for production of the artificial variant, wherein the mutant polynucleotide was obtained by the methods described herein; and (b) recovering the artificial variant.

In the production methods of the present invention, the cells are cultivated in a nutrient medium suitable for production of the artificial variant using methods well known in the art. For example, the cell may be cultivated by shake flask cultivation, and small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the artificial variant is secreted into the nutrient medium, the polypeptide can be recovered directly from the medium. If the artificial variant is not secreted, it can be recovered from cell lysates.

The artificial variants may be detected using methods known in the art that are specific for the variants. These detection methods may include use of specific antibodies, formation of an enzyme product, or disappearance of an enzyme substrate. For example, an enzyme assay may be used to determine the activity of the polypeptide as described herein. A multiplicity of assays are available and known in the art. For examples see Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, D.C. (1994)) or by Thomas D. Brock in Biotechnology: A Textbook of Industrial Microbiology, Second Edition, Sinauer Associates, Inc., Sunderland, Mass. (1989).

The resulting artificial variant may be recovered using methods known in the art. For example, the variant may be recovered from the nutrient medium by conventional procedures including, but not limited to, centrifugation, filtration, extraction, spray-drying, evaporation, or precipitation.

The artificial variants of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, *e.g.,* Protein Purification, J.-C. Jason and Lars Ryden, editors, VCH Publishers, New York, 1989) to obtain substantially pure variants.

The present invention is further described by the following examples which should not be construed as limiting the scope of the invention.

### Examples

### Yeast strain

*Saccharomyces cerevisiae* JG169 (MATα, ura3-52, leu2-3, pep4-1137, his3Δ2, prb1::leu2, and Δpre1::his3) was used for expression of the beta-glucosidase random insertional library.

### Example 1: Construction of pSATe111 Saccharomyces cerevisiae expression vector

A 2,605 bp DNA fragment comprising the region from the ATG start codon to the TAA stop codon of an *Aspergillus oryzae* beta-glucosidase coding sequence (SEQ ID NO: 1 for cDNA sequence and SEQ ID NO: 2 for the deduced amino acid sequence) was amplified by PCR from pJaL660 (WO 2002/095014) as template with primers 992127 (sense) and 992328 (antisense) shown below.
992127: 5'-GCAGATCTACCATGAAGCTTGGTTGGATCGAG-3' (SEQ ID NO: 3)
992328: 5'-GCCTCGAGTTACTGGGCCTTAGGCAGCGAG-3' (SEQ ID NO: 4)
Primer 992127 has an upstream *Bgl* II site and primer 992328 has a downstream *Xho* I site.

The amplification reactions (50 µl) were composed of 1X PCR buffer containing MgCl₂ (Roche Applied Science, Manheim, Germany), 0.25 mM dNTPs, 50 µM primer 992127, 50 µM primer 992328, 80 ng of pJaL660, and 2.5 units of Pwo DNA Polymerase (Roche Applied Science, Manheim, Germany). The reactions were incubated in an Eppendorf Mastercycler 5333 (Eppendorf Scientific, Inc., Westbury, NY) programmed for 1 cycle at 94°C for 5 minutes followed by 25 cycles each at 94°C for 60 seconds, 55°C for 60 seconds, and 72°C for 120 seconds (10 minute final extension).

The PCR product was then subcloned into the PCR-Blunt II-TOPO vector using the PCR-Blunt II-TOPO Cloning Kit (Invitrogen, Carlsbad, CA) following the manufacturer's instructions to generate pSATe101 (Figure 1). Plasmid pSATe101 was digested with *Bgl* II and *Xho* I to liberate the beta-glucosidase gene. The reaction products were isolated on a 1.0% agarose gel using 40 mM Tris-acetate-1 mM EDTA (TAE) buffer where a 2.6 kb product band was excised from the gel and purified using a QlAquick Gel Extraction Kit (QIAGEN Inc., Valencia, CA) according to the manufacturer's instructions.

The 2.6 kb PCR product was digested and cloned into the *Bam* HI and *Xho* I sites of the copper inducible 2 µm yeast expression vector pCu426 (Labbe and Thiele, 1999, Methods Enzymol. 306: 145-53) to generate pSATe111 (Figure 2).

### Example 2: Construction of Aspergillus oryzae beta-glucosidase entry vector

The *Aspergillus oryzae* beta-glucosidase gene was amplified by PCR using plasmid pSATe111 as a template. The following primers were used to amplify the beta-glucosidase gene with the desired restriction sites (the restriction recognition sites are italicized and the beta-glucosidase coding sequence is underlined).
Forward primer Jal660_BG_Sal1_F:
5'-GCACGC*GTCGAC* ACCATGAAGCTTGGTTGGATCGAG-3' (SEQ ID NO: 5)
Reverse primer aBGXho.1A
5'-GATGCACATGACTCGAGTTACTGG-3' (SEQ ID NO: 6)

The amplification reactions (50 µl) were composed of 1X PCR buffer containing MgCl₂, 0.2 mM dNTPs, 50 pM each primer, 50 ng of pSATE111, and 2.5 units of Herculase DNA Polymerase (Stratagene Inc., La Jolle, CA). The reactions were incubated in an Eppendorf Mastercycler 5333 programmed for 1 cycle at 95°C for 3 minutes followed by 30 cycles each at 95°C for 30 seconds, 55°C for 30 seconds, and 72°C for 90 seconds (5 minute final extension).

The PCR product (approximately 2.6 kb) was purified using a MiniElute™ Kit (QIAGEN Inc., Valencia, CA) according to the manufacture's instructions.

The PCR product was restriction digested with *Sal* I and *Xho* I and ligated into pENTR 1A (Invitrogen, Carlsbad, CA) which was also digested with *Sal* I and *Xho* I to generate pAJF-1 (Figure 3). The ligation reaction was carried out using a Rapid Ligation Kit (Roche Applied Science, Manheim, Germany). Plasmid pAJF-1 contains a kanamycin resistance gene, a pUC origin of replication for maintenance in *E. coli,* and two att sites flanking the beta-glucosidase gene for LR Clonase™ mediated Gateway recombination.

### Example 3: Construction of an Aspergillus oryzae beta-glucosidase destination vector

The entry vector pAJF-1 containing the *Aspergillus oryzae* beta-glucosidase gene was used to generate the destination vector pAJF-2 through recombination with plasmid pYESDEST-52 (Invitrogen, Carlsbad, CA) mediated by Gateway LR Clonase™ (Invitrogen, Carlsbad, CA) according to the manufacturer's instructions. The Gateway LR recombination reaction (20 µl) was composed of 300 ng of pAJF-1, 300 ng pYESDEST-52, 1X reaction buffer (Invitrogen, Carlsbad, CA), and 4 µl of LR Clonase™. The reaction was incubated for 21 hours at 25°C. Proteinase K (2 µg/µl) was added and the reaction was incubated for 10 minutes at 37°C. An aliquot (1 µl) from this reaction was used to transform *E*. *coli* Top 10 competent cells (Invitrogen, Carlsbad, CA). Ampicillin selection and sequence analysis of a colony isolate confirmed proper insertion of the *Aspergillus oryzae* beta-glucosidase gene in pYESDEST-52. This plasmid, identified as pAJF-2 (Figure 4), contains the GAL1 promoter for inducible gene expression in *Saccharomyces cerevisiae,* the beta-lactamase gene coding for ampicillin resistance in *E. coli,* the pUC ori for replication in *E. coli*, the URA3 *Saccharomyces cerevisiae* auxotrophic selection marker, and the *Saccharomyces cerevisiae* 2µ origin of replication. Plasmid pAJF-2 was used as a wild-type control for comparison with pAJF-2 transposon insertion libraries.

### Example 4: Random Insertional library generation

The Entranceposon M1-Cam^{R} (Finnzymes Oy, Espoo, Finland) and the Mutation Generation System™ (MGS™, Finnzymes Oy, Espoo, Finland) were used to generate transposon insertions in plasmid pAJF-1 according to the manufacturer's instructions.

The Entranceposon M1-Cam^{R} utilizes the bacteriophage Mu transposase to insert an artificial transposon at random positions within a target DNA population (Mizuuchi, 1992, Annual Review of Biochemistry 61: 1011-1051; Haapa et al., 1999, Nucleic Acids Research 27: 2727-2784). The artificial 1.254 kb transposon used in this system contains the following components: 44 bp 5' and 3' conserved tandem inverted repeats which act as recognition sites for the Mu transposase, *Not* I sites located within the inverted repeats that are used for transposon removal and self-ligation, and internal to these repeats is the coding sequence for a chloramphenicol selection marker. After insertion, the transposon can subsequently be removed using the restriction enzyme *Not* I followed by self-ligation of the backbone which results in a 15 bp in-frame insertion. Ten of 15 bps inserted originate from the inverted repeat sequence that flanks the transposon. The other 5 bp are a result of duplication of the target site that occurs upon integration. The five amino acid insert can be translated into three different peptide combinations based on the insertion frame. In one frame three of the five amino acids are alanines, which is a desired outcome for less deleterious changes to the overall structure of a protein.

Five different transposition reactions were performed with the following modifications from the Finnzymes protocol: (1) 200 ng of pAJF-1; (2) 100 ng of pAJF-1; (3) 100 ng of pAJF-1, 2 µl of MuA transposase (Finnzymes Oy, Espoo, Finland), and incubated at 30°C for 2 hours; (4) 1 µg of pAJF-1; and (5) 1 µg of pAJF-1, 2 µl of MuA transposase, and incubated at 30°C for 2 hours. Each reaction (20 µl) consisted of the indicated quantity of DNA, 1X MuA transposase buffer, 100 ng of Entranceposon M1-Cam^{r}, and 1 µl of MuA transposase (Finnzymes Oy, Espoo, Finland). The reactions were incubated for 1 hour at 30°C and then 10 minutes at 75°C.

Competent *E. coli* Top10 cells (Invitrogen, Carlsbad, CA) were transformed with 5 µl of each of the transposition reactions. Transformants were selected on LB agar plates supplemented with 50 µg of kanamycin per ml and 10 µg of chloramphenicol per ml grown overnight at 37°C. The resistant colonies were rinsed off the plates and DNA was isolated using a Plasmid Midi-Prep Kit (QIAGEN Inc., Valencia, CA). Five separate libraries were generated from the five different transposon reactions.

Approximately 20,000 pAJF-1 clones containing a transposon in the plasmid were isolated from the five transposon reactions using dual antibiotic selection (*i*.*e*., the entry vector encodes kanamycin resistance and the transposon chloramphenicol).

Following transposon mutagenesis, the mutated beta-glucosidase genes from the transposon-containing entry vector library were transferred to the Gateway yeast destination vector pYESDEST-52. LR Clonase™ was used to carry out the Gateway transfer reaction according to the manufacturer's instructions with the following modifications: 300 ng of destination vector, 300 ng of entry vector, and the reaction time was extended to 21 or 25 hours.

Competent *E. coli* Top10 cells were transformed with 1 to 2 µl of the Gateway reaction. Transformants were selected on LB agar plates supplemented with 100 µg of ampicillin per ml and 10 µg of chloramphenicol per ml grown overnight at 37°C. Resistant colonies were rinsed off the plates and DNA was isolated using a QIAGEN Plasmid Midi-Prep Kit. Approximately 26,000 clones were isolated. A small portion of the transformation was also plated onto LB agar plates supplemented with 100 µg of ampicillin per ml. A portion of these colonies were then patched onto LB agar plates supplemented with 100 µg of ampicillin per ml and 10 µg of chloramphenicol per ml to determine the approximate number of pENTR1A clones containing a transposon located outside of the beta-glucosidase coding region. As a negative control the Gateway reaction was carried out without the entry vector to determine the ampicillin resistant background generated from the destination vector.

The results showed that between 43 and 67% of the clones subjected to transposon mutagenesis contained a gene-directed insertion, representing about 10,000 clones. The negative control reaction showed that only three colonies were ampicillin resistant, resulting in a very low background of vector alone from the Gateway reaction.

The inserted transposon was subsequently removed from the library to leave a 15 bp insertion. This was accomplished by collecting library colonies into a single pool and utilizing a QlAfilter Midi Plasmid Kit (QIAGEN Inc., Valencia, CA) to isolate library plasmid DNA. The restriction endonuclease *Not* I was utilized to excise the Mu transposase recognition sites and the chloramphenicol selectable marker. Agarose gel (0.8%) electrophoresis using TAE buffer was used to identify the library plasmid void of the artificial transposon. This backbone fragment was gel purified using a QiaQuick Gel Purification Kit (QIAGEN Inc., Valencia, CA) and religated using a Rapid Ligation Kit (Roche Applied Science, Manheim, Germany) according to the manufacturer's instructions with the following modifications: 100 ng or 20 ng of vector DNA was used, and the reaction time was extended to 30 minutes at 16°C. Competent *E. coli* Top10 cells were transformed with 5 µl of the ligation reaction.

Transformants were selected on LB agar plates supplemented with 100 µg of ampicillin per ml grown overnight at 37°C. Approximately, 66,000 clones were isolated, representing 10,000 independent insertion events. From this library, 96 resistant clones were patched onto LB agar plates supplemented with 100 µg of ampicillin per ml and 10 µg of chloramphenicol per ml to obtain an estimate of the number of clones containing the full transposon insert. Only 1 transformant survived dual selection, suggesting that less than 2% of the library contained the full transposon insertion.

For characterization and sequencing purposes, the 50 ampicillin resistant colonies were grown overnight in LB medium and DNA was obtained using a QIAGEN QlAfilter Midi Plasmid Kit.

### Example 5: Random Insertional library characterization

The beta-glucosidase insertional mutants from the final transposon libraries after the transposon was removed were sequenced to determine the position and type of insertion resulting. DNA sequencing was performed on an ABI3700 (Applied Biosystems, Foster City, CA) using dye terminator chemistry (Giesecke et al., 1992, Journal of Virol. Methods 38: 47-60). Sequences were assembled using phred/phrap/consed (University of Washington, Seattle WA) with sequence specific primers. Fifty clones were sequenced, revealing that 47 (94%) of the clones contained inserts, 2 (4%) lacked inserts, and 1 (2%) contained the entire transposon. Of the 47 clones with inserts, 3 of them had only 14 bp inserts resulting in frame shift mutations. All three of these mutants had the same deletion in the 10 bp sequence that is left from the transposon inverted repeat sequence. Of the 47 clones with inserts, 41 clones were unique. Eleven clones in total resulted from identical insertions at 5 different sites (Figure 5). However, there were no obvious hot spots where preferential insertion seemed to be occurring. The 15 bp insert can result in different amino acid combinations based on the frame of insertion. Based on the 41 unique clones, 16 (39%) of the inserts occurred in the first frame, 14 (34%) in the second, and 11 (27%) in the third.

### Example 6: Expression of the beta-glucosidase random Insertional library In Saccharomyces cerevisiae

To study the beta-glucosidase phenotype of the 41 variants containing inserts described in Example 5, plasmid DNA from all 41 variants was used to transform *Saccharomyces cerevisiae* JG169. The YeastMaker Yeast Transformation System 2 (Clontech Laboratories, Inc., Palo Alto, CA) was used for transformation according to the manufactures instructions.

Selection and induction of the beta-glucosidase insertional mutant transformants was accomplished by plating the transformation on galactose induction medium. Galactose induction medium was composed per liter of 6.7 g of yeast nitrogen base with ammonium sulfate, 5 g of casamino acids, 20 g of agar, and 100 ml of 0.5 M sodium succinate pH 5.0, brought to 860 ml with deionized water, autoclaved for 25 minutes, and cooled to 55°C. After cooling, the following filter sterilized supplements were added: 40 ml of 50% glucose (final 2%), 100 ml of 20% D(+)-galactose (final 2%), and 0.2 ml of 500 mg/ml 5-bromo-4-chloro-3-indolyl-beta-D-glucopyranoside (X-glc) (final 100 mg/l) in DMSO (final 0.02% vol/vol). Yeast colonies were grown for 3 to 5 days at 30°C. Colonies producing active beta-glucosidase turned blue after incubation due to beta-glucosidase hydrolysis of X-glc. Qualitative beta-glucosidase activity was estimated by visual intensity of the blue color and size of the colony.

The beta-glucosidase activity for these clones fell into 7 color/size categories: dark blue (tiny colonies, WT like) 13%, dark blue (medium sized) 10%, blue (medium sized), light blue (medium sized) 19%, very light blue (medium sized) 4%, mixture of white and blue 4%, and no color 38%. These phenotypes were matched on the insertion distribution map (Figure 6).

### Example 7: Codon triplet substitution - using Bsg I and Btg ZI

A polypeptide encoding a substitution variant of the glucoamylase from *Talaromyces emersonii*, T-AMG, was constructed according to the present invention. The experiments performed are outlined below:
(1) Transposons with kanamycin resistance were inserted into plasmid pMiBg235 yielding libraries of about 1 x 10⁶ transformants.
(2) Experiments where transformants were plated out on either ampicillin or kanamycin plates showed 100 times more colonies on ampicillin plates, which indicated a high probability for only one transposon per gene.
(3) Plasmid preparations of pooled transformants showed that only DNA with the gene coding for kanamycin resistance was obtained.
(4) Restriction with enzymes flanking the gene of interest yielded four strong bands on agarose gels: a fragment containing the gene, gene with transposon, vector minus gene, and vector minus gene with transposon.
(5) The cloning steps showed relatively high transformation rates between 600,000 to 12 x 10⁶ transformants.
(6) Sequence analysis of resulting plasmids from each cloning step showed the expected restrictions and finally the desired substitutions (see below).

DNA fragment manufacture. Enzymes and a transposon kit ('Mutation Generation System') were purchased from Finnzymes Oy, Espoo, Finland, 'PCR Polishing Kit' was from Stratagene Corp., La Jolla, CA, and oligos were obtained from DNA Technology, Arhus, Denmark.

Two oligos were designed with various restriction sites (see Figure 7A for details).
tcgagatcgaacagcggccgcatcgcagctggcaggtacggatcgatcctagtaagcca (SEQ ID NO: 13)

A *Not* I-*Not* I DNA fragment was synthezised by PCR with the designed oligos using the commercial transposon ENTRANCEPOSON™ (Finnzymes Oy, Espoo, Finland) (M1-Kanamycin) as template (the sequence of the transposon is shown in SEQ ID NO: 9). To achieve high transformation rates, the synthesized fragment with the outside cutter recognition sites and the three random or partially random base pairs 'NNN' (N indicates 25% of T, C ,G, and A) was first subcloned (6,400 transformants). Subsequently, the fragment was introduced into the inserted transposon in the gene of interest, replacing most of the inserted transposon in the process.

Cloning of T-AMG gene. Plasmid pSteD202 is an episomal expression vector based on the very well-known inducible yeast expression vector pYES2, wherein the gal4 promoter of pYES2 was replaced by a constitutive triose phophate isomerase (TPI) promoter, using standard procedures. The TPI promoter ensures constitutive expression of the gene, when the gene of interest is cloned downstream the TPI promoter. The vector comprises the URA3 marker, a gene of the synthetic pathway for uracil, encoding oritidine 5'-decarboxylase which allows for selection on minimal medium. The vector further contains the 2My origin of DNA replication. An ampicillin resistance gene is conveniently used for selection in E. coli.

The cDNA of the T-AMG gene encoding the amyloglucosidase from *Talaromyces emersonii* was cloned into the yeast/*E. coli* shuttle vector pSteD202 as a *Hin*dIII/*Xba*I PCR fragment to yield the vector pSteD226. pSteD202 is derived from the yeast expression vector pYES 2.0 (Invitrogen, UK and Kofod et al., 1994, J. Biol. Chem. 269: 29182-29189). Both pSteD202 and pSteD226 replicate in *E. coli* and *S. cerevisiae*.

Plasmid pMiBg235 is identical to pSteD226, except that one Al restriction site and three *Btg* ZI restriction sites present in pSteD226 have been removed to facilitate the use of these 'outside cutting' restriction enzymes in the cloning steps of the invention.

Insertion of transposon. The Finnzymes 'Mutation Generation System' kit was used for random insertion of a transposon into pMiBg235, which contains the gene coding for an amyloglucosidase (AMG) from a *Talaromyces* species, denoted T-AMG. Three hundred and ten ng of pMiBg235 were mixed with 100 ng of Entranceposon (M1-Kanamycin) (Finnzymes Oy, Espoo, Finland), 1 µl of MuA transposase, and 4 µl of the manufacturer's 5X MuA reaction buffer in a total volume of 20 µl. The transposition reaction was allowed to proceed for 60 minutes at 30°C and the MuA transposase was subsequently inhibited by incubation at 75°C for 10 minutes.

Plasmid DNA was isolated and purified into a volume of 15 µl, 1 or 3 µl thereof was then electrotransformed into competent *E. coli* cells according to standard procedures, and transformants were spread out onto LB plates supplemented with 10 µg/ml kanamycin to yield 16,000 and 65,000 kanamycin resistant transformants, respectively. The procedure was repeated to yield a total number of about 1 x 10⁶ transformants. Transposon containing plasmid DNA was purified from overnight incubations of selected transformed *E. coli* cells in LB medium supplemented with 100 µg of ampicillin and 10 µg of kanamycin per ml.

Isolation of T-AMG genes with transposons. In order to isolate T-AMG genes with transposons, 10 µg of plasmid was restricted with *Pac* I and *Xba* I, which should result in four DNA fragments: the original vector, the T-AMG gene fragment, plus vector- and T-AMG gene fragments with inserted transposon. The T-AMG gene DNA fragment with the transposon inserted was isolated by agarose gel electrophoresis and cloned back into *Pac* I and *Xba* I digested pMiBg235 vector; 600,000 kanamycin-resistant transformants were obtained.

Introduction of DNA fragment with outside cutter sites. DNA-fragments flanked with outside cutters (Figure 7) were cloned into the library of T-AMG genes (with transposons) using the two flanking *Not* I-sites of the inserted transposon: 10 µg of plasmid DNA of the T-AMG (with transposons) was digested with *Not* I and the vector and T-AMG fragments were isolated from the transposon fragment and ligated to the *Not* I restricted PCR-fragments; 600,000 kanamycin-resistant transformants were obtained.

Trimming flanking site by *Bsg* I restriction. A fragment containing one of the *Not* I-sites and parts of the neighboring duplicated target site was digested from the construct with *Bsg* I and the vector/T-AMG DNA-fragment purified on an agarose gel. The remaining sticky-ends were blunt-ended by PCR polishing, removing all five base pairs in the duplicated target site. The three random or partially random base pairs were brought next to the coding sequence of T-AMG by ligation of the two blunt ends of the vector/T-AMG DNA-fragment. The circularized vector was then transformed into *E. coli* yielding 5.6 x 10⁶ transformants.

Trimming flanking site by *Btg* ZI and *Pvu* II restriction. A fragment containing one of the *Not*I-sites and parts of the neighboring duplicated target site was digested with *Btg* ZI and *Pvu* II, and the vector/T-AMG DNA-fragments were isolated from an agarose gel. The remaining sticky-ends were blunt-ended by PCR polishing by filling in basepair 1 and 2 of the duplicated target site. A *Bfu* Al site was brought into a position close to the coding sequence of T-AMG by subsequent ligation of the two blunt ends of the vector/T-AMG DNA-fragment. The circulated vector was transformed into *E. coli* yielding 8 x 10⁵ transformants.

Excision of transposon by *Bfu* Al restriction. The remaining fragment was excised by digestion with *Bfu* Al and the linearized vector was purified from an agarose gel. The sticky-ends were then PCR polished and the vector was relegated. The position of the *Bfu* Al site with respect to basepair 1 and 2 of the duplicated target site was designed so that the *Bfu* Al restriction in this step would bring the random or partially random codon-triplet 'NNN' into position next to base pair 1 and 2 after the religation, thereby replacing base pair 3, 4 and 5 of the duplicated target site. The circularized vector was transformed into *E. coli* yielding 12 x 10⁶ transformants.

Sequence analysis. DNA-sequence analysis of three different resulting variants of the *Talaromyces* amyloglucosidase yielded the following amino acid substitutions:

| | | |
|---|---|---|
| **Variant 1: Q82W** | Position: | |
| Amino acid sequence of wt: | N-term. | |
| Coding sequence of wt: | | |
| Coding sequence of variant 1: | N-term. | |
| | | |
| **Variant 2: Q81G** | Position: | |
| Amino acid sequence of wt: | N-term. | |
| Coding sequence of wt: | | |
| Coding sequence of variant 2: | | |
| | N-term. | |
| | | |
| **Variant 3: S165P.** | Position: | |
| Amino acid sequence of wt: | N-term. | |
| Coding sequence of wt: | | |
| Coding sequence of variant 3: | | |
| | N-term. | |

### Example 8: Codon triplet substitution using Bsg I and Acu I

A polypeptide encoding a substitution variant of a maltogenic amylase from *Bacillus stearothermophilus* was constructed according to the present invention. The experiments performed are outlined below:
(1) Transposons with kanamycin resistance were inserted into plasmid pMiBg242 yielding libraries of about 1 x 10⁶ transformants.
(2) Experiments where transformants were plated out on either ampicillin or kanamycin plates showed 100 times more colonies on ampicillin plates, which indicated a high probability for only one transposon per gene.
(3) Plasmid preparations of pooled transformants showed that only DNA with the gene coding for kanamycin resistance was obtained.
(4) Restriction with enzymes flanking the gene of interest yielded four strong bands on agarose gels: a fragment containing the gene, gene with transposon, vector minus gene, and vector minus gene with transposon.
(5) The cloning steps showed relatively high transformation rates between 600.000 to 12 x 10⁶ transformants.
(6) Sequence analysis of resulting plasmids from each cloning step showed the expected restrictions and finally the wanted substitutions (see text below).

DNA fragment manufacture. Enzymes and transposon kit ('Mutation Generation System') were purchased from Finnzymes Oy, Espoo, Finland, 'PCR Polishing Kit' was from Stratagene Corp., La Jolla, CA, and oligos were obtained from DNA Technology, Arhus, Denmark.

Two oligos were designed with various restriction sites (see Figure 8A):

tcgagatcgaacagcggccgctggacttcagacggatcgatcctagtaagcca (SEQ ID NO: 12)

A PCR-fragment was synthesized with the designed oligos using the commercial transposon ENTRANCEPOSON™ (M1-Kanamycin) as template (the sequence of the transposon is shown in SEQ ID NO: 9). To achieve high transformation rates, the synthesized fragment with the outside cutter recognition sites and the three random or partially random base pairs 'NNN' (N indicates 25% of T, C, G and A) was first subcloned (6,400 transformants). Subsequently, the *Not* I-digested PCR-fragment was introduced into the *Not* I-sites of the previously inserted transposon in the gene of interest, effectively replacing most of the inserted transposon in the process (see Figure 8B).

Cloning of T-AMG gene. The *Acu* I sites of pMiBg235 vector described above were removed to yield the vector pMiBg231 to facilitate the use of this 'outside cutting' restriction enzyme in the cloning steps of the invention. The cDNA of a gene encoding a maltogenic amylase from *Bacillus stearothermophilus* was cloned into the yeast/*E. coli* shuttle vector pMiBg231 as a *Pac* I/*Xba* I PCR fragment without *Acu* I sites to yield the vector pMiBg242.

Insertion of transposon. The Finnzymes 'Mutation Generation System' kit was used for random insertion of a transposon into plasmid DNA containing the gene coding for the maltogenic amylase. A total of 310 ng of pMiBg242 was mixed with 100 ng of Entranceposon (M1-Kanamycin), 1 µl of MuA transposase, and 4 µl of the manufacturer's 5x MuA reaction buffer in a total volume of 20 µl. The transposition reaction was allowed to proceed for 60 minutes at 30°C and the MuA transposase was subsequently inhibited by incubation at 75°C for 10 minutes.

Plasmid DNA was isolated and purified into a volume of 15 µl, 1 or 3 µl thereof was then electrotransformed into competent *E. coli* cells according to standard procedures, and transformants were spread out on LB-kanamycin plates (10 µg/ml) yielding 16,000 and 65,000 kanamycin resistent transformants, respectively. The procedure was repeated yielding a total number of about 1 x 10⁶ transformants. Transposon containing plasmid DNA was purified from overnight incubations of selected transformed *E*. *coli* cells in LB-ampicillin (100 µg/ml) and kanamycin (10 µg/ml) medium.

Isolation of genes with transposons. In order to isolate genes with transposons, 10 µg of plasmid was restricted with *Pac* I and *Xba* I, which should result in four DNA fragments: the original vector, the gene fragment, plus vector- and gene fragments with inserted transposon. The maltogenic amylase encoding gene fragment with the transposon inserted was isolated by agarose gel electrophoresis and cloned back into *Pac* I and *Xba* I restricted pMiBg242 vector. More than 500,000 kanamycin-resistant transformants were obtained.

Introduction of DNA fragment with outside cutter sites. *Not* I-digested DNA-fragments flanked with outside cutters were introduced into the library of maltogenic amylase genes (with transposons) in the two flanking *Not* I-sites of the inserted transposon: 10 µg of plasmid DNA of the amylase encoding gene (with transposons) was cut with *Not* I and the vector- and gene-fragments were isolated from the transposon fragment and ligated to the *Not* I restricted PCR-fragments. More than 500,000 kanamycin-resistant transformants were obtained.

Trimming flanking site bv *Bsg* I restriction. A fragment containing one of the *Not* I-sites and parts of the neighbouring duplicated target site was digested from the construct with *Bsg* I and the vector/gene-fragment purified on agarose gel. The remaining sticky-ends were blunt-ended by PCR polishing, removing all five base pairs in the duplicated target site. The three random or partially random base pairs were brought next to the coding sequence of the maltogenic amylase gene by ligation of the two blunt ends of the vector/gene-fragment. The circularized vector was then transformed into *E*. *coli* yielding more than 1 x 10⁶ transformants.

Trimming flanking site and excision of transposon by *Acu* I restriction. The remaining transposon fragment was excised by restriction with *Acu* I of two *Acu* I sites at each end of the inserted transposon and the linearized vector was purified from an agarose gel. The sticky-ends were then PCR polished and the vector was religated. The design of the position of one of the *Acu* I sites with respect to basepair 1 and 2 of the duplicated target site was done so that the *Acu* I restriction in this step would bring the random or partially random codon-triplet 'NNN' into position next to base pair 1 and 2 after the religation, thereby replacing base pair 3, 4 and 5 of the duplicated target site. The circularized vector was transformed into *E. coli* yielding more than 1 x 10⁶ transformants.

Sequence analysis. DNA-sequence analysis of three different resulting variants gave following amino acid substitutions:

| | | |
|---|---|---|
| **Variant 1: D326T** | Position: | |
| Amino acid sequence of wt: | N-term. | |
| Coding sequence of wt: | | |
| Coding sequence of variant 1: | | |
| | N-term. | |
| **Variant 2: K340I** | Position: | |
| Amino acid sequence of wt: | N-term. | |
| Coding sequence of wt: | | |
| Coding sequence of variant 2: | | |
| | N-term. | |

### Example 9: Codon triplet deletion

A polypeptide encoding a deletion variant of a maltogenic amylase from *Bacillus stearothermophilus* was constructed according to the present invention. The experiments showed that it was possible to insert a transposon into the gene of interest and that transposon could be excised to provide one, two or three deleted codon triplets in the gene. The experiments performed are outlined below:
(1) Transposons with kanamycin resistance were inserted into plasmid pMiBg242 yielding libraries of about 1 x 10⁶ transformants.
(2) Experiments where transformants were plated out on either ampicillin or kanamycin plates showed 100 times more colonies on ampicillin plates, which indicated a high probability for only one transposon per gene.
(3) Plasmid preparations of pooled transformants showed that only DNA with the gene coding for kanamycin resistance was obtained.
(4) Restriction with enzymes flanking the gene of interest yielded four strong bands on agarose gels: a fragment containing the gene, gene with transposon, vector minus gene and vector minus gene with transposon.
(5) The cloning steps showed relatively high transformation rates between 600.000 to 12x10⁶ transformants.
(6) Sequence analysis of resulting plasmids from each cloning step showed the expected restrictions and finally the wanted deletions (see text below).

DNA fragment manufacture. Enzymes and a transposon kit ('Mutation Generation System') were purchased from Finnzymes Oy, Espoo, Finland, 'PCR Polishing Kit' was from Stratagene Corp., La Jolla, CA, and oligos were obtained from DNA Technology, Århus, Denmark.

Two oligos to obtain one deleted codon triplets were designed with various restriction sites (see Figure 9A for details):
atcgagctcagcggccgcctgcaccggatgatccagttcgatttattc (SEQ ID NO: 19)
tcgagatcgaacagcggccgcaaggaactgcacacggatcgatcctagtaagcca (SEQ ID NO: 15)

To obtain two or three deleted codon triplets instead of just one, two oligos were designed with various restriction sites to replace SEQ ID NO 9, respectively, in the following strategy:
Two deleted codon triplets:
tcgagatcgaacagcggccgcaagctgcacacggatcgatcctagtaagcca (SEQ ID NO: 20)
Three deleted codon triplets:
tcgagatcgaacagcggccgcctgcacacggatcgatcctagtaagcca (SEQ ID NO: 21)

A *Not* I-*Not* I DNA fragment was synthesized by PCR with the designed oligos using the commercial transposon ENTRANCEPOSON™ as template (the sequence of the transposon is shown in SEQ ID NO: 9). To achieve high transformation rates, the synthesized fragment with the outside cutter recognition sites was first subcloned (7,000 transformants). Subsequently, the fragment was cloned into the inserted transposon in the gene of interest, replacing most of the inserted transposon in the process.

Cloning of amylase gene. The *Acu* I sites of pMiBg235 described above were removed to yield the vector pMiBg231 to facilitate the use of these 'outside cutting' restriction enzymes in the cloning steps of the invention. The cDNA of the gene encoding the maltogenic amylase from *Bacillus stearothermophilus* was cloned into the yeast/*E. coli* shuttle vector pMiBg231 as a *Pac* I/*Xba* I PCR fragment without *Acu* I sites to yield the vector pMiBg242.

Insertion of transposon. The Finnzymes 'Mutation Generation System' kit was used for random insertion of transposon into plasmid DNA containing the gene coding for the maltogenic amylase. A total of 310 ng of pMiBg242 was mixed with 100 ng of Entranceposon (M1-Kanamycin), 1 µl of MuA transposase, and 4 µl of the manufacturer's 5x MuA reaction buffer in a total volume of 20 µl. The transposition reaction was allowed to proceed for 60 minutes at 30°C, and the MuA transposase was subsequently inhibited by incubation at 75°C for 10 minutes.

Plasmid DNA was isolated and purified into a volume of 15 µl, 1 or 3 µl thereof was then electrotransformed into competent *E. coli* cells according to standard procedures, and transformants were spread out on LB-kanamycin plates (10 µg/ml) yielding 16,000 and 65,000 kanamycin resistent transformants, respectively. The procedure was repeated yielding a total number of about 1 x 10⁶ transformants. Transposon-containing plasmid DNA was purified from overnight incubations of selected transformed *E*. *coli* cells in LB-ampicillin (100 µg/ml) and kanamycin (10 µg/ml) medium.

Isolation of genes with transposons. In order to isolate genes with transposons, 10 µg of the above purified plasmid was digesed with *Pac* I and *Xba* I, which should result in four DNA fragments: the original vector, the gene fragment, plus vector- and gene fragments with inserted transposon. The amylase encoding gene fragment with the transposon inserted was isolated by agarose gel electrophoresis and cloned back into *Pac* I and *Xba* I digested pMiBg242. Approximately, 600, 000 kanamycin-resistant transformants were obtained.

Introduction of DNA fragment with outside cutter sites. *Not* I-digested DNA-fragments flanked with outside cutters were introduced into the library of maltogenic amylase genes (with transposons) in the two flanking *Not* I-sites of the inserted transposon: 10 µg of plasmid DNA of the amylase encoding gene (with transposons) was digested with *Not* I and the vector- and gene-fragments were isolated from the transposon fragment and ligated to the *Not* I restricted PCR-fragments. More than 600,000 kanamycin-resistant transformants were obtained.

Trimming flanking sites and excision of transposon by *Bsg* I restriction. The transposon fragment and parts of the flanking sequences were digested from the construct with *Bsg* I of two *Bsg* I sites at each end of the inserted transposon and the linearized vector was purified from an agarose gel. The position of one of the *Bsg* I sites was designed so that *Bsg* I restriction would remove all of the five duplicated base pairs plus two more base pairs (right site in Figure 9B). The position of the other *Bsg* I site was designed so that *Bsg* I restriction would remove base pair 5 (left site in Figure 9). The sticky-ends were then PCR polished and the vector was religated so that a triplet of basepairs was deleted. The circularized vector was then transformed into *E. coli* yielding more than 1 x 10⁶ transformants.

Sequence analysis. DNA-sequence analysis of six different resulting variants gave following DNA and amino acid deletions ('D260*' means residue D260 is deleted):
One deleted codon triplets:

| | | |
|---|---|---|
| **Variant 1: D260*.** | Position: | |
| Amino acid sequence of wt: | N-term. | |
| Coding sequence of wt: | | |
| Coding sequence of variant 1: | | |
| | N-term. | |

Two deleted codon triplets were also constructed:

| | | |
|---|---|---|
| **Variant 2: V129*, P130*** Position: | | |
| Amino acid sequence of wt: | N-term. | |
| Coding sequence of wt: | | |
| Coding sequence of variant 2: | | |
| | N-term. | |
| | | |
| **Variant 3: N131*, H132*** Position: | | |
| Amino acid sequence of wt: | N-term. | |
| Coding sequence of wt: | | |
| Coding sequence of variant 3: | | |
| | N-term. | |
| | | |
| **Variant 4: S476T, V477*, A478*** | Position: | |
| Amino acid sequence of wt: | N-term. | |
| Coding sequence of wt: | | |
| Coding sequence of variant 4: | | |
| | N-term. | |

Three deleted codon triplets were also constructed:

| | | |
|---|---|---|
| **Variant 5: V254*, G255*, E256** | Position: | |
| Amino acid sequence of wt: | N-term. | |
| Coding sequence of wt: | | |
| Coding sequence of variant 5: | | |
| | N-term. | |
| | | |
| **Variant 6: H267Q, L268*, E269*, K270*.** | | |
| | Position: | |
| Amino acid sequence of wt: | N-term. | |
| Coding sequence of wt: | | |
| Coding sequence of variant 6: | | |
| | N-term. | |

## Claims

1. A method of producing at least one mutant of a polynucleotide, the method comprising the steps of:
(a) isolating a first library of constructs, wherein each construct comprises a first selectable marker, a polynucleotide, an inserted artificial transposon comprising at least two restriction endonuclease recognition sites and a second selectable marker, and a first recombination site flanking the 5' end of the polynucleotide and a second recombination site flanking the 3' end of the polynucleotide, wherein the artificial transposon has inserted at one or more random sites within the constructs, and wherein the first library is selected using the first and second selectable markers in a first host cell;
(b) isolating a second library of constructs by introducing the first library of constructs into a vector comprising a third selectable marker and a first recombination site and a second recombination site to facilitate site-specific recombination of the first recombination site flanking the 5' end of the polynucleotide and the second recombination site flanking the 3' end of the polynucleotide in the first library of constructs with the first recombination site and the second recombination site of the vector and by selecting the second library of constructs using the second and third selectable markers in a second host cell;
(c) isolating an insertion library containing at least one substitution, deletion, or insertion of at least one nucleotide in each polynucleotide of the second library of constructs by removing all, essentially all, or a portion of the inserted artificial transposon from the second library of constructs through restriction endonuclease digestion of the at least two restriction endonuclease recognition sites leaving at least one substitution, deletion, or insertion of at least one nucleotide in the polynucleotide; self-ligating the restriction endonuclease digested fragments; and selecting the insertion library using the third selection marker in a third host cell; and
(d) isolating at least one mutant of the polynucleotide from the insertion library, wherein the isolated mutant comprises at least one substitution, deletion, or insertion of at least one nucleotide in the polynucleotide.

2. The method of claim 1, wherein the polynucleotide encodes a polypeptide.

3. The method of claim 2, wherein the polypeptide is an antibody, hormone, enzyme, receptor, reporter, or selectable marker.

4. The method of claim 3, wherein the enzyme is an oxidoreductase, transferase, hydrolase, lyase, isomerase, or ligase.

5. The method of claim 1, wherein the polynucleotide is a control sequence.

6. The method of claim 5, wherein the control sequence is a leader, polyadenylation sequence, propeptide sequence, promoter, signal peptide sequence, or transcription terminator.

7. The method of claim 1, wherein the polynucleotide is an origin of replication.

8. The method of claim 1, wherein the construct is a plasmid or vector.

9. The method of any of claims 1-8, wherein the artificial transposon comprises 5' and 3' conserved tandem inverted repeats which act as recognition sites for a transposase; a selectable marker gene located within the transposon sequence; and at least two restriction endonuclease recognition sites for transposon and selectable marker removal, and for introduction of one or more substitutions, deletions, or insertions, and self-ligation.

10. The method of claim 9, wherein the at least two restriction endonuclease recognition sites comprise one or more inside cutter recognition sequences.

11. The method of claim 10, wherein restriction of the one or more inside cutter recognition sites results in one or more insertions of the polynucleotide.

12. The method of claim 9, wherein the at least two restriction endonuclease recognition sites comprise one or more outside cutter recognition sites.

13. The method of claim 12, wherein restriction of the one or more outside cutter recognition sites results in one or more deletions of the polynucleotide.

14. The method of claim 12, wherein restriction of the one or more outside cutter recognition sites results in one or more substitutions of the polynucleotide.

15. A method of producing at least one polynucleotide encoding at least one variant of a parent polypeptide, the method comprising the steps of:
(a) providing a nucleic acid construct comprising a polynucleotide encoding the parent polypeptide, into which polynucleotide has been inserted a heterologous polynucleotide fragment, wherein said fragment comprises at least two restriction endonuclease recognition sites;
(b) restricting the nucleic acid construct with at least two corresponding restriction endonucleases, if necessary in separate individual steps of restricting, PCR-polishing, and ligating, wherein all or essentially all of the inserted heterologous fragment is excised from the construct and at least one nucleotide triplet is deleted, inserted, or substituted in the encoding polynucleotide in the process, whereby at least one polynucleotide encoding at least one variant of the parent polypeptide is produced.

16. The method of claim 15, wherein the parent polypeptide is an enzyme, preferably an oxidoreductase, transferase, hydrolase, lyase, isomerase, or ligase.

17. The method of claim 15 or 16, wherein the construct is a DNA plasmid.

18. The method of any of claims 15-17, wherein the heterologous polynucleotide fragment comprises a transposon.

19. The method of any of claims 15-18, wherein the heterologous polynucleotide fragment comprises at least one random or partially random codon triplet 'NNN'.

20. The method of any of claims 15-19, wherein the at least two restriction endonuclease recognition sites comprise one or more outside cutter restriction endonuclease recognition site.

21. The method of any of claims 15-20, wherein the at least two restriction endonuclease recognition sites comprise one or more outside cutter restriction endonuclease recognition site, and wherein restriction with the one or more corresponding outside cutter endonuclease results in one or more cut in the polynucleotide outside of the inserted heterologous polynucleotide fragment.

22. The method of any of claims 15-21, wherein the at least two restriction endonuclease recognition sites comprise two or more different outside cutter restriction endonuclease recognition sites.

23. The method of any of claims 15-22, wherein the heterologous polynucleotide fragment comprises a selection marker, preferably an antibiotic resistance marker.

24. The method of any of claims 15-23, wherein the heterologous polynucleotide fragment comprises a polynucleotide having the sequence shown in SEQ ID NO: 10.

## Patentansprüche

1. Verfahren zum Herstellen mindestens einer Mutante von einem Polynukleotid, wobei das Verfahren die Schritte umfasst:
(a) Isolieren einer ersten Bibliothek von Konstrukten, wobei jedes Konstrukt einen ersten Selektionsmarker, ein Polynukleotid, ein eingefügtes künstliches Transposon, das mindestens zwei Restriktionsendonuklease-Erkennungsstellen und einen zweiten Selektionsmarker umfasst, und eine erste Rekombinationsstelle, flankierend das 5'-Ende des Polynukleotids, und eine zweite Rekombinationsstelle, flankierend das 3'-Ende des Polynukleotids, umfasst, wobei sich das künstliche Transposon an einer oder mehreren zufälligen Stellen innerhalb des Konstrukts eingefügt hat, und wobei die erste Bibliothek ausgewählt wird unter Verwendung des ersten und zweiten Selektionsmarkers in einer ersten Wirtszelle;
(b) Isolieren einer zweiten Bibliothek von Konstrukten durch Einführen der ersten Bibliothek von Konstrukten in einen Vektor, umfassend einen dritten Selektionsmarker und eine erste Rekombinationsstelle und eine zweite Rekombinationsstelle, zum Ermöglichen einer ortsspezifischen Rekombination der ersten Rekombinationsstelle, flankierend das 5'-Ende des Polynukleotids, und der zweiten Rekombinationsstelle, flankierend das 3'-Ende des Polynukleotids, in der ersten Bibliothek von Konstrukten mit der ersten Rekombinationsstelle und der zweiten Rekombinationsstelle des Vektors, und durch Auswählen der zweiten Bibliothek von Konstrukten unter Verwendung des zweiten und dritten Selektionsmarkers in einer zweiten Wirtszelle;
(c) Isolieren einer Insertionsbibliothek, enthaltend mindestens eine Substitution, Deletion oder Insertion von mindestens einem Nukleotid in jedem Polynukleotid der zweiten Bibliothek von Konstrukten durch Entfernen aller, im Wesentlichen aller oder einem Teil der eingefügten künstlichen Transposons aus der zweiten Bibliothek von Konstrukten durch Restriktionsendonuklease-Verdau der mindestens zwei Restriktionsendonuklease-Erkennungsstellen, die mindestens eine Substitution, Deletion oder Insertion von mindestens einem Nukleotid in dem Polynukleotid hinterlassen; Selbstligieren der Restriktions-Endonuklease-verdauten Fragmente; und Auswählen der Insertionsbibliothek unter Verwendung des dritten Selektionsmarkers in einer dritten Wirtszelle; und
(d) Isolieren mindestens einer Mutante des Polynukleotids aus der Insertionsbibliothek, wobei die isolierte Mutante mindestens eine Substitution, Deletion oder Insertion von mindestens einem Nukleotid in dem Polynukleotid umfasst.

2. Verfahren nach Anspruch 1, wobei das Polynukleotid ein Polypeptid kodiert.

3. Verfahren nach Anspruch 2, wobei das Polypeptid ein Antikörper, Hormon, Enzym, Rezeptor, Reporter oder Selektionsmarker ist.

4. Verfahren nach Anspruch 3, wobei das Enzym eine Oxidoreduktase, Transferase, Hydrolase, Lyase, Isomerase oder Ligase ist.

5. Verfahren nach Anspurch 1, wobei das Polynukleotid eine Kontrollsequenz ist.

6. Verfahren nach Anspruch 5, wobei die Kontrollsequenz ein Leader, eine Polyadenylierungssequenz, eine Propeptidsequenz, ein Promotor, eine Signalpeptidsequenz oder ein Transkriptionsterminator ist.

7. Verfahren nach Anspruch 1, wobei das Polynukleotid ein Replikationsursprung ist.

8. Verfahren nach Anspruch 1, wobei das Konstrukt ein Plasmid oder ein Vektor ist.

9. Verfahren nach einem beliebigen der Ansprüche 1-8, wobei das künstliche Transposon 5'- und 3'-konservierte tandeminvertierte Wiederholungen, die als Erkennungsstellen für eine Transposase agieren; ein Selektionsmarkergen, das innerhalb der Transposonsequenz lokalisiert ist; und mindestens zwei Restriktionsendonuklease-Erkennungsstellen zur Entfernung des Transposon und des Selektionsmarkers, und zur Einführung von einer oder mehreren Substitutionen, Deletionen oder Insertionen und zur Selbstligation umfasst.

10. Verfahren nach Anspruch 9, wobei die mindestens zwei Restriktionsendonuklease-Erkennungsstellen eine oder mehrere innen schneidende Erkennungssequenzen umfassen.

11. Verfahren nach Anspruch 10, wobei Restriktion der einen oder mehreren innen schneidenden Erkennungsstellen in einer oder mehreren Insertionen des Polynukleotids resultiert / resultieren.

12. Verfahren nach Anspruch 9, wobei die mindestens zwei Restriktionsendonuklease-Erkennungsstellen eine oder mehrere außerhalb schneidende Erkennungsstellen umfasst.

13. Verfahren nach Anspruch 12, wobei Restriktion der einen oder mehreren außerhalb schneidenden Erkennungsstellen in einer oder mehreren Deletionen des Polynukleotids resultiert / resultieren.

14. Verfahren nach Anspruch 12, wobei Restriktion der einen oder mehreren außerhalb schneidenden Erkennungsstellen in einer oder mehreren Substitutionen des Polynukleotids resultiert / resultieren.

15. Verfahren zum Herstellen mindestens eines Polynukleotids, kodierend mindestens eine Variante eines Ausgangspolypeptids, wobei das Verfahren die Schritte umfasst:
(a) Bereitstellen eines Nukleinsäurekonstrukts, umfassend ein Polynukleotid, kodierend das Ausgangspolypeptid, in welches Polynukleotid ein heterologes Polynukleotidfragment eingefügt worden ist, wobei das Fragment mindestens zwei Restriktionsendonuklease-Erkennungsstellen umfasst;
(b) Schneiden des Nukleinsäurekonstrukts mit mindestens zwei entsprechenden Restriktionsendonukleasen, falls notwendig in getrennten einzelnen Schritten des Schneidens, PCR-Polierens, und Ligierens, wobei das gesamte oder im wesentlichen Gesamte des eingefügten heterologen Fragments aus dem Konstrukt ausgeschnitten wird und mindestens ein Nukleotidtriplet in dem kodierenden Polynukleotid in dem Verfahren deletiert, insertiert oder substituiert wird, wobei mindestens ein Polynukleotid, das mindestens eine Variante des Ausgangspolypeptids kodiert, hergestellt wird.

16. Verfahren nach Anspruch 15, wobei das Ausgangspolypeptid ein Enzym, vorzugsweise eine Oxidoreduktase, Transferase, Hydrolase, Lyase, Isomerase oder Ligase ist.

17. Verfahren nach Anspruch 15 oder 16, wobei das Konstrukt ein DNA-Plasmid ist.

18. Verfahren nach einem beliebigen der Ansprüche 15-17, wobei das heterologe Polynukleotidfragment ein Transposon umfasst.

19. Verfahren nach einem beliebigen der Ansprüche 15-18, wobei das heterologe Polynukleotidfragment mindestens ein zufälliges oder teilweise zufälliges Codontriplet 'NNN' umfasst.

20. Verfahren nach einem beliebigen der Ansprüche 15-19, wobei die mindestens zwei Restriktionsendonuklease-Erkennungsstellen eine oder mehrere außerhalb schneidende Restriktionsendonuklease-Erkennungsstellen umfasst.

21. Verfahren nach einem beliebigen der Ansprüche 15-20, wobei die mindestens zwei Restriktionsendonuklease-Erkennungsstellen eine oder mehrere außerhalb schneidende Restriktionsendonuklease-Erkennungsstellen umfasst, und wobei Schneiden mit der einen oder mehreren entsprechenden außerhalb schneidenden Endonuklease in einem oder mehreren Schnitten in dem Polynukleotid außerhalb des eingefügten heterologen Polynukleotidfragments resultiert / resultieren.

22. Verfahren nach einem beliebigen der Ansprüche 15-21, wobei die mindestens zwei Restriktionsendonuklease-Erkennungsstellen zwei oder mehrere unterschiedliche außerhalb schneidende Restriktionsendonuklease-Erkennungsstellen umfassen.

23. Verfahren nach einem beliebigen der Ansprüche 15-22, wobei das heterologe Polynukleotidfragment einen Selektionsmarker, vorzugsweise einen Antibiotika-Resistenzmarker umfasst.

24. Verfahren nach einem beliebigen der Ansprüche 15-23, wobei das heterologe Polynukleotidfragment ein Polynukleotid mit der in SEQ ID NO: 10 gezeigten Sequenz umfasst.

## Revendications

1. Procédé de production d'au moins un mutant d'un polynucléotide, le procédé comprenant les étapes consistant à :
(a) isoler une première banque de constructions, dans laquelle chaque construction comprend un premier marqueur sélectionnable, un polynucléotide, un transposon artificiel inséré comprenant au moins deux sites de reconnaissance d'endonucléase de restriction et un deuxième marqueur sélectionnable, et un premier site de recombinaison flanquant l'extrémité 5' du polynucléotide et un second site de recombinaison flanquant l'extrémité 3' du polynucléotide, où le transposon artificiel a été inséré au niveau d'un ou plusieurs sites aléatoires au sein des constructions, et où la première banque est sélectionnée en utilisant les premier et deuxième marqueurs sélectionnables dans une première cellule hôte ;
(b) isoler une seconde banque de constructions en introduisant la première banque de constructions dans un vecteur comprenant un troisième marqueur sélectionnable et un premier site de recombinaison et un second site de recombinaison pour faciliter la recombinaison spécifique du site du premier site de recombinaison flanquant l'extrémité 5' du polynucléotide et le second site de recombinaison flanquant l'extrémité 3' du polynucléotide dans la première banque de constructions avec le premier site de recombinaison et le second site de recombinaison du vecteur et en sélectionnant la seconde banque de constructions en utilisant les deuxième et troisième marqueurs sélectionnables dans une deuxième cellule hôte ;
(c) isoler une banque d'insertion contenant au moins une substitution, une délétion ou une insertion d'au moins un nucléotide dans chaque polynucléotide de la seconde banque de constructions en éliminant la totalité, pratiquement la totalité, ou une partie du transposon artificiel inséré à partir de la seconde banque de constructions par l'intermédiaire d'une digestion avec une endonucléase de restriction des au moins deux sites de reconnaissance d'endonucléase de restriction en laissant au moins une substitution, une délétion ou une insertion d'au moins un nucléotide dans le polynucléotide ; auto-ligaturer les fragments digérés par les endonucléases de restriction ; et sélectionner la banque d'insertion en utilisant le troisième marqueur de sélection dans une troisième cellule hôte ; et
(d) isoler au moins un mutant du polynucléotide à partir de la banque d'insertion, où le mutant isolé comprend au moins une substitution, une délétion ou une insertion d'au moins un nucléotide dans le polynucléotide.

2. Procédé selon la revendication 1, dans lequel le polynucléotide code pour un polypeptide.

3. Procédé selon la revendication 2, dans lequel le polypeptide est un anticorps, une hormone, une enzyme, un récepteur, un rapporteur ou un marqueur sélectionnable.

4. Procédé selon la revendication 3, dans lequel l'enzyme est une oxydoréductase, une transférase, une hydrolase, une lyase, une isomérase ou une ligase.

5. Procédé selon la revendication 1, dans lequel le polynucléotide est une séquence de contrôle.

6. Procédé selon la revendication 5, dans lequel la séquence de contrôle est une séquence de tête, une séquence de polyadénylation, une séquence de propeptide, un promoteur, une séquence de peptide signal ou un terminateur de la transcription.

7. Procédé selon la revendication 1, dans lequel le polynucléotide est une origine de réplication.

8. Procédé selon la revendication 1, dans lequel la construction est un plasmide ou un vecteur.

9. Procédé selon l'une quelconque des revendications 1 à 8, dans lequel le transposon artificiel comprend des répétitions inversées en tandem conservées en 5' et 3' qui agissent comme des sites de reconnaissance pour une transposase ; un gène de marqueur sélectionnable localisé au sein de la séquence du transposon ; et au moins deux sites de reconnaissance d'endonucléase de restriction pour l'élimination du transposon et du marqueur sélectionnable, et pour l'introduction d'une ou plusieurs substitutions, délétions, ou insertions, et une auto-ligature.

10. Procédé selon la revendication 9, dans lequel les au moins deux sites de reconnaissance d'endonucléase de restriction comprennent une ou plusieurs séquences de reconnaissance de coupure interne.

11. Procédé selon la revendication 10, dans lequel la restriction d'un ou plusieurs sites de reconnaissance de coupure interne entraîne une ou plusieurs insertions du polynucléotide.

12. Procédé selon la revendication 9, dans lequel les au moins deux sites de reconnaissance d'endonucléase de restriction comprennent un ou plusieurs sites de reconnaissance de coupure externe.

13. Procédé selon la revendication 12, dans lequel la restriction d'un ou plusieurs sites de reconnaissance de coupure externe entraîne une ou plusieurs délétions du polynucléotide.

14. Procédé selon la revendication 12, dans lequel la restriction d'un ou plusieurs sites de reconnaissance de coupure externe entraîne une ou plusieurs substitutions du polynucléotide.

15. Procédé de production d'au moins un polynucléotide codant pour au moins un variant d'un polypeptide parent, le procédé comprenant les étapes consistant à :
(a) fournir une construction d'acide nucléique comprenant un polynucléotide codant pour le polypeptide parent, dans lequel polynucléotide a été inséré un fragment de polynucléotide hétérologue, où ledit fragment comprend au moins deux sites de reconnaissance d'endonucléase de restriction ;
(b) restreindre la construction d'acide nucléique avec au moins deux endonucléases de restriction correspondantes, si nécessaire dans des étapes individuelles séparées de restriction, polissage par PCR, et ligature, où la totalité ou pratiquement la totalité du fragment hétérologue inséré est excisée à partir de la construction et au moins un triplet de nucléotides est délété, inséré, ou substitué dans le polynucléotide codant dans le processus, par lequel au moins un polynucléotide codant pour au moins un variant du polypeptide parent est produit.

16. Procédé selon la revendication 15, dans lequel le polypeptide parent est une enzyme, de préférence une oxydoréductase, une transférase, une hydrolase, une lyase, une isomérase, ou une ligase.

17. Procédé selon la revendication 15 ou 16, dans lequel la construction est un plasmide d'ADN.

18. Procédé selon l'une quelconque des revendications 15 à 17, dans lequel le fragment de polynucléotide hétérologue comprend un transposon.

19. Procédé selon l'une quelconque des revendications 15 à 18, dans lequel le fragment de polynucléotide hétérologue comprend au moins un codon triplet 'NNN' aléatoire ou partiellement aléatoire.

20. Procédé selon l'une quelconque des revendications 15 à 19, dans lequel les au moins deux sites de reconnaissance d'endonucléase de restriction comprennent un ou plusieurs sites de reconnaissance d'endonucléase de restriction de coupure externe.

21. Procédé selon l'une quelconque des revendications 15 à 20, dans lequel les au moins deux sites de reconnaissance d'endonucléase de restriction comprennent un ou plusieurs sites de reconnaissance d'endonucléase de restriction de coupure externe, et dans lequel la restriction avec l'une ou plusieurs endonucléases de coupure externe correspondantes entraîne une ou plusieurs coupures dans le polynucléotide en dehors du fragment de polynucléotide hétérologue inséré.

22. Procédé selon l'une quelconque des revendications 15 à 21, dans lequel les au moins deux sites de reconnaissance d'endonucléase de restriction comprennent deux ou plus de sites de reconnaissance d'endonucléase de restriction de coupure externe.

23. Procédé selon l'une quelconque des revendications 15 à 22, dans lequel le fragment de polynucléotide hétérologue comprend un marqueur de sélection, de préférence un marqueur de résistance à un antibiotique.

24. Procédé selon l'une quelconque des revendications 15 à 23, dans lequel le fragment de polynucléotide hétérologue comprend un polynucléotide ayant la séquence représentée par SEQ ID NO: 10.
